# EUROPEAN PATENT SPECIFICATION

(11) **EP 0 918 069 B1**
(45) Date of publication and mention of the grant of the patent: **09.04.2008**
(21) Application number: 98923063.6
(22) Date of filing: 01.06.1998
(51) Int. Cl.: C08L 101/10, A61K 8/00, C08F 230/08

(54) **COMPOSITION CONTAINING A COPOLYMER COMPRISING REACTIVE SILYL GROUPS AND APPLICATION METHODS OF THE SAME**
ZUSAMMENSETZUNG EIN COPOLYMER ENTHALTEND DAS REAKTIVE SILYL-GRUPPEN AUFWEIST SOWIE VERFAHREN ZUR VERWENDUNG
COMPOSITIONS CONTENANT UN COPOLYMERE AUX GROUPES SILYLES REACTIFS ET METHODES DE LEUR EMPLOI

(30) Priority: 30.05.1997 JP 15767597; 30.05.1997 JP 15767697; 30.05.1997 JP 15767797; 29.08.1997 JP 24954797; 29.08.1997 JP 24954897; 20.10.1997 JP 30644297; 20.02.1998 JP 5575198; 20.02.1998 JP 5575298; 20.02.1998 JP 5575398
(43) Date of publication of application: 26.05.1999
(73) Proprietor: SHISEIDO COMPANY LIMITED, Chuo-ku, Tokyo 104-8010 (JP)
(72) Inventor: MIYAZAWA, Kazuyuki Shiseido Research Center (1), Yokohama-shi Kanagawa 223-8553 (JP); YANAKI, Toshio, Kohoku-ku Yokohama-shi Kanagawa 223-8553 (JP); MATSUZAKI, Fumiaki, Kohoku-ku Yokohama-shi Kanagawa 223-8553 (JP)
(74) Representative: Henkel, Feiler & Hänzel
(86) International application number: PCT/JP1998/002407
(87) International publication number: WO 1998/054255

(56) References cited:
- EP-A- 0 297 505
- EP-A- 0 348 946
- EP-A- 0 412 707
- EP-A- 0 690 109
- EP-A- 0 824 127
- WO-A-94/00520
- GB-A- 2 192 400
- JP-A- 2 008 285
- JP-A- 3 128 312
- JP-A- 4 321 618
- JP-A- 5 000 924
- JP-A- 5 017 324
- JP-A- 5 214 676
- JP-A- 5 239 435
- JP-A- 6 009 332
- JP-A- 6 166 613
- JP-A- 7 097 770
- JP-A- 8 012 901
- JP-A- 8 253 539
- JP-A- 9 110 632
- JP-A- 9 328 677
- JP-A- 59 203 738

## Description

### FIELD OF THE INVENITON

The present invention relates to a copolymer containing reactive silyl groups, a composition comprising the same, and a treating method with the same. Particularly, the present invention relates to: a copolymer, which forms a strong coat of the cross-linked copolymer having resistance to washing on the surface of hair, skin or other materials to useful effects in the fields of cosmetics and the others such as modifying effect of the nature of hair, improving effect of makeup retention or water-repellent effect; a composition comprising the same; and a treating method with the same.

### BACKGROUND OF THE INVENTION

A numerous film-forming ingredients are used in the fields of cosmetics and the others for the purpose of giving various effects.

However, there are very few film-forming ingredients that have an useful effect as a cosmetic ingredient and that maintain the effect against washing with shampoo or detergent.

Also, in the other fields, for example, in the field of water-repellents that give water-repellency to fiber, paper or the other materials, it is expected to improve resistance to washing.

EP-A-0 297 505 discloses an antifouling paint that contains as its essential components an antifoulant and a polymer of at least one of monomers A, A' and A" represented by the following general formulae (a), (b) and (c), respectively, and/or a copolymer composed of at least one of the monomers A, A' and A" and one or more vinyl polymerizable monomers B that are copolymerizable with said monomers A, A' and A": 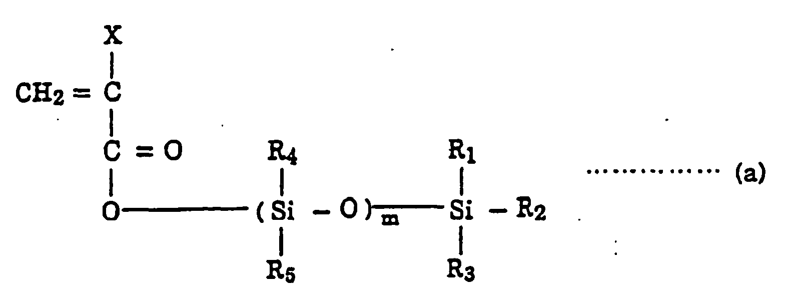 wherein X is a hydrogen atom or a methyl group; m is a real number of 1 or more; R₁-R₅ each is a group selected from the group consisting of an alkyl group, an alkoxy group, a phenyl group, a substituted phenyl group, a phenoxy group and a substituted phenoxy group, in which R₁-R₅ may be the same or different; 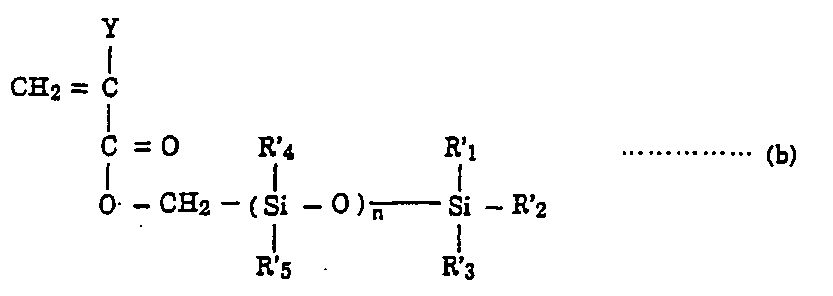 wherein Y is a hydrogen atom or a methyl group; n is 0 or more; R'₁-R'₅ each is a group selected from the group consisting of an alkyl group, an alkoxy group, a phenyl group, a substituted phenyl group, a phenoxy group and a substituted phenoxy group, in which R'₁-R'₅ may be the same or different; and 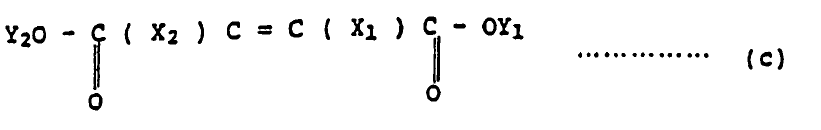 wherein X₁ and X₂ each is a hydrogen atom or a methyl group which may be either cis or trans form; one of Y₁ and Y₂ is an organic group represented by the following general formula (1) : 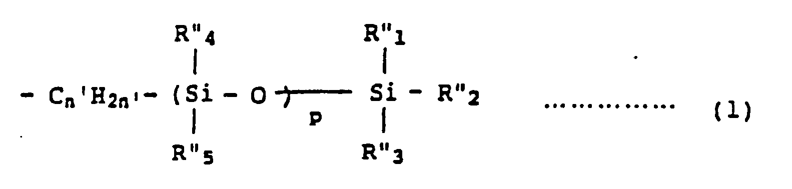 wherein n' is an integer of 0 or 1; p is 0 or more; R"₁-R"₅, which may be the same or different, each is a group selected from the group consisting of an alkyl group, an alkoxy group, a phenyl group, a substituted phenyl group, a phenoxy group, a substituted phenoxy group and an organosiloxane group; with the other being a hydrogen atom, an alkyl group, a phenyl group, a substituted phenyl group or an organic group represented by formula (1).

EP-A-0 690 109 discloses an aqueous primer composition comprising a copolymer wherein a cationic monomer, a specific alkoxysilane monomer, a silane macromonomer and an ethylenically unsaturated monomer are contained in aqueous medium. For this reason, regardless of the alkalinity of the substrate, the aqueous primer composition can form a highly water-resistant, alkali-resistant, tough film.

EP-A-0 412 707 discloses hair care compositions which provide improved styling and hair conditioning properties. These compositions comprise from about 0.1% to about 10.0% of a specifically-defined silicone copolymer and from about 0.5% to about 99.5% of a carrier suitable for application to hair.

### SUMMARY OF THE INVENTION

The present invention has been achieved in view of the foregoing problems of the prior art. The first object of the present invention is to find a compound that can form a coat and to provide a composition comprising the compound, wherein said coat demonstrates useful effects as a cosmetic ingredient, e.g., hair nature modifying effect, make-up retention improving effect of hair colors or make-up cosmetics, or skin-protecting activity and these effects are maintained due to high resistance to washing.

Also, the second object of the present invention is to find a compound that can form a coat, which is excellent in water-repellency and resistance to washing, on the materials to be treated such as fiber, and to provide a composition comprising the compound.

As a result of the diligent studies performed by the inventors so as to achieve the foregoing objects, it has been found that a strong coat of a cross-linked copolymer that is excellent in resistance to washing can be formed when a copolymer containing a silyl group that one or more reactive functional group is bonded to (e.g., a monomer having trifunctional silyl group), as a constitutive monomer is cross-linked with hydrolysis on the material to be treated such as hair or skin.

Also, it has been ascertained that such cross-linked coat has hair nature modifying effect and skin protecting effect and these effects are maintained without loss even when shampoo is repeatedly used.

Also, it has been ascertained that the cross-linked coat formed by such copolymer ameliorates resistance to washing of make-up cosmetics such as temporary hair color, acidic hair dye, mascara or nail enamel and improves make-up retention.

Also, it has been ascertained that a cosmetic pack comprising the copolymer has high skin-cleaning ability and is excellent in applicability, strippability and film strength thereof.

Further, the present inventors have found that a coat which has high water-repellency, unsusceptibility to fouling and suitability for sizing and is excellent in resistance to washing can be formed even when the copolymer is treated with respect to the other materials such as fiber, paper or glass. Accordingly, the present invention has been accomplished.

Namely, the present invention provides a composition comprising a copolymer containing silyl groups wherein each silyl group has at least one reactive functional group bonded thereto,
wherein said copolymer comprises monomer (A) shown by the following Formula (I): 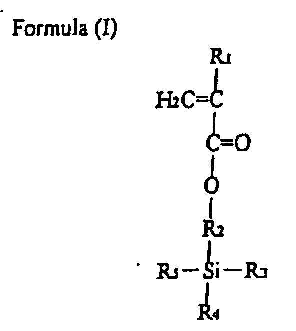 wherein R₁ is a hydrogen atom or methyl group; R₂ is an alkylene group having 1-6 carbon atoms; and R₃, R₄ and R₅ each is a reactive functional group which can cross-link molecules of the copolymer by hydrolyzing,
monomer B shown by the following Formula (II): 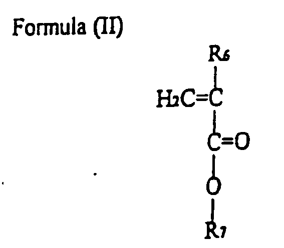 wherein R₆ is a hydrogen atom or methyl group; and R₇ is an alkyl group having 1-18 carbon atoms,
monomer (C) shown by the following Formula (III): 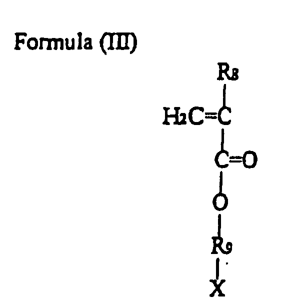 wherein R₈ is a hydrogen atom or methyl group; R₉ is an alkylene group having 1-6 carbon atoms; and X is a group expressed by any of the following Formulae (TV) to (VI): 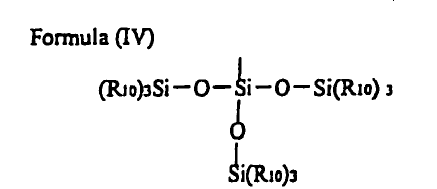 wherein R₁₀ is an alkyl group having 1-6 carbon atoms; 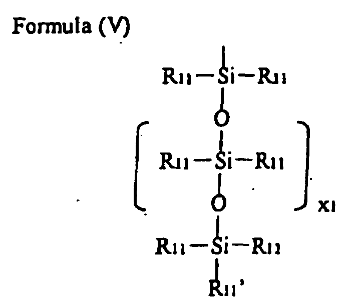 wherein R₁₁ and R₁₁' each is an alkyl group having 1-6 carbon atoms; and X₁ is an integer of positive number; and 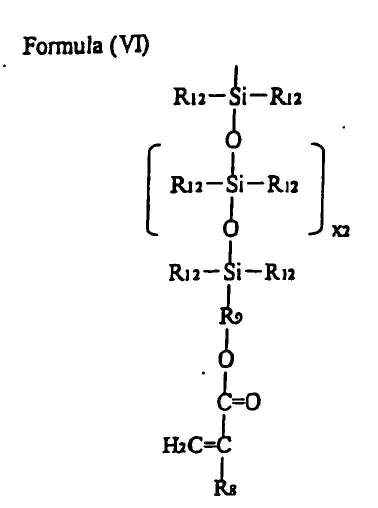 wherein R₈ and R₉ are the same as defined in Formula (III); R₁₂ is an alkyl group having 1-6 carbon atoms; and x₂ is an integer of positive number, and
monomer (D) shown by the following formula (VII): 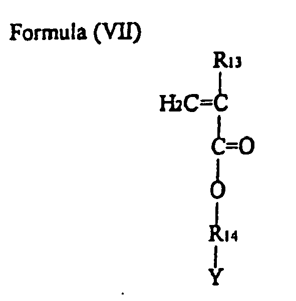 wherein R₁₃ is a hydrogen atom or methyl group; R₁₄ is an alkylene group having 1-6 carbon atoms; and Y is a group expressed by -N+(R₁₅)₃ or -N(R₁₅)₂, wherein R₁₅ is an alkyl group having 1-6 carbon atoms.

It is preferable that the percentage of monomer (A) is 25 to 99 wt% in the copolymer.

Also, it is preferable that the percentage of monomer (B) is 1 wt% or more in the copolymer.

Also, it is preferable that the percentage of monomer (C) is 1 wt% or more in the copolymer.

It is preferable that the percentage of monomer (D) is within the range of 1 to 100 parts by weight with respect to 100 parts by weight of a combination of monomer (A), monomer (B) and monomer (C) in the copolymer.

Also, it is preferable that each of R₃, R₄, and R₅ is an alkoxy group having 1-6 carbon atoms.

It is preferable that the composition further comprises a pigment and/or an acidic dye.

Also, it is preferable that the composition further comprises a siliconized polysaccharide.

The present invention further provides a copolymer comprising monomer (A) shown by Formula (I) as defined above, monomer (B) shown by Formula (II) as defined above, monomer (C) shown by Formula (III) as defined above and monomer (D) shown by Formula (VII) as defined above, as constitutive monomers.

In a preferred embodiment in the copolymer of the present invention, each of R₃, R₄ and R₅ is an alkoxy group having 1 to 6 carbon atoms.

Furthermore, the present invention provides a use of the composition according to the present invention for coating a surface; a use of a composition according to the present invention for modifying the nature of hair; a use of a composition according to the present invention as a cosmetic pack; and a use of a composition according to the present invention as a water-repellent.

In addition, in a use of the composition according to the present invention as a water-repellent preferably the percentage of monomer (A) is 25 to 85 wt% in the copolymer; and preferably the percentages of monomer (B) and monomer (C) are 5 wt% or more and 1 wt% or more in the copolymer, respectively.

Furthermore, the present invention provides a use of a composition according to the present invention as a fibre-treating composition.

In said use preferably the percentage of monomer (A) is 25 to 85 wt% in the copolymer, and preferably the percentages of monomer (B) and monomer (C) are 5 wt% or more and 1 wt% or more in the copolymer, respectively.

A coat-forming method in accordance with the present invention is characterized by comprising a step of: hydrolyzing the composition as defined above on a material to be treated to cross-link molecules of the copolymer.

In the method of the present invention, it is preferable that the material to be treated is human hair or epidermis.

Also, in the method of the present invention, it is preferable that the material to be treated is fiber, paper or glass.

A method for a pack in accordance with the present invention is characterized by comprising steps of hydrolyzing the composition according to the present invention on skin to cross-link molecules of the copolymer; and peeling the composition from the skin after drying.

### BRIEF DESCRIPTION OF THE DRAWINGS

Fig. 1 shows one example of cross-linking reaction of the copolymer in accordance with the present invention.
Fig. 2 is a diagram showing NMR spectrum of the copolymer in accordance with one example of the present invention.

### BEST MODE FOR CARRYING OUT THE INVENTION

In the following, the embodiment for carrying out the present invention will be explained.

The copolymer comprised in the composition of the present invention contains silyl groups having at least one reactive functional group bonded thereto.

Examples of the copolymer include a copolymer having tri-functional silyl groups.

The copolymer of the present invention is a copolymer comprising monomer (A) shown by Formula (I) as a constitutive monomer.

Monomer (A) is an acrylate or methacrylate comprising a trifunctional silyl group - SiR₃R₄R₅. R₁ is a hydrogen atom or methyl group in Formula (I). Also, R₂ is an alkylene group having 1 to 6 carbon atoms and preferably, is propylene group.

R₃, R₄, and R₅ each is a reactive functional group, which can cross-link molecules of the copolymer of the present invention due to forming a siloxane bond Si-O-Si by hydrolysis. Examples of such functional group include a hydrogen atom, alkoxy group, halogen atom, acyloxy group and amino group. An alkoxy group having 1 to 6 carbon atoms is preferable in terms of stability of the copolymer, safety of byproduct formed by hydrolysis, reactivity of the cross-linking reaction mentioned later, and the like. Methoxy or ethoxy group is more preferable. Also, R₃, R₄, and R₅ each may be the same or different. In the copolymer of the present invention, at least one of monomer (A) is used as a constitutive monomer.

The copolymer of the present invention comprises in addition to monomer (A) containing the reactive silyl group, monomer (B) shown by the foregoing Formula II as a constitutive monomer.

Monomer (B) is an alkyl acrylate or alkyl methacrylate. In Formula (II), R₆ is a hydrogen atom or methyl group. R₇ is a straight, branched or cyclic alkyl group having 1 to 18 carbon atoms. However, R₇ is preferably an alkyl group having 1 to 6 carbon atoms and, more preferably, methyl group. In the copolymer of the present invention, at least one of the foregoing monomer (B) can be used as a constitutive monomer.

Furthermore, the copolymer of the present invention further comprises monomer (C) of the foregoing Formula (III) as a constitutive monomer. Monomer (C) is siloxane-containing (meth)acrylate. R₈ is a hydrogen atom or methyl group in Formula (III). R₉ is an alkylene group having 1 to 6 carbon atoms and, preferably, ethylene, propylene or 2-hydroxypropylene group. X is a siloxane expressed by any of the foregoing Formulae (IV) to (VI). R₁₀, R₁₁, R₁₁' and R₁₂ each is a straight or branched alkyl group having 1 to 6 carbon atoms or phenyl group in Formulae (IV) to (VI). Although R₁₀, R₁₁ and R₁₂ each exist plurally in these formulae, they may be the same or different from each other. Methyl group is preferable as R₁₀, R₁₁ and R₁₂. Butyl group is preferable as R₁₁'. A molecular weight of monomer (C) is 1,000 to 100,000 and, preferably, 2,000 to 20,000 in the case where X is Formula (V) or (VI).

The copolymer of the present invention further comprises amine-containing (meth)acrylate monomer (D) shown by the foregoing Formula (VII). R₁₃ is a hydrogen atom or methyl group in Formula (VII). R₁₄ is an alkylene group having 1 to 6 carbon atoms and, preferably, ethylene or propylene group. Y is a group shown by -N⁺(R15)3 or -N(R₁₅)₂, and R₁₅ is an alkyl group having 1 to 6 carbon atoms. Also, Y may be a salt whose counter ion is halogen, inorganic acid, organic acid or the like, in the case
where Y is the group of -N⁺(R₁₅)₃.

Also, it is possible that the copolymer according to the present invention comprises monomer (E) which is different from the above-mentioned monomers (A) to (D), as a constitutive monomer within a range that the effect of the present invention is not spoiled.

A preferable example of the copolymer comprised in the composition of the present invention can be, for example, a copolymer shown by the following Formula (X): 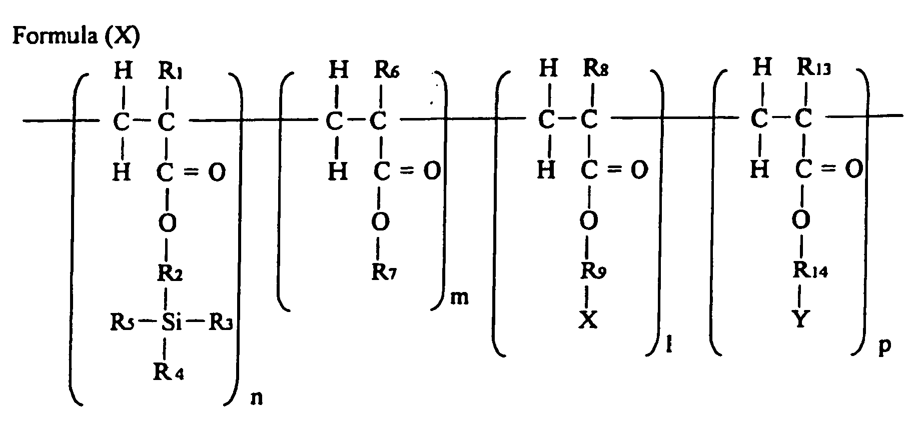 wherein R₁, R₂, R₃, R₄, R₅, R₆, R₇, R₈, R₉, R₁₃, R₁₄, X and Y are the same as defined above, and n, m, 1, and p are molar ratios of monomers (A), (B), (C) and (D), respectively

The copolymer in accordance with the present invention can be obtained by polymerizing the foregoing monomers by means of a known polymerization method such as solution polymerization method, emulsion polymerization method or bulk polymerization method. For example, in case of solution polymerization method, the copolymer of the present invention can be obtained by dissolving each monomer into a solvent with the desired monomer ratio, adding a radical polymerization initiator under nitrogen atmosphere, and heating with stirring.

As a solvent used in polymerization, it is possible to use any solvent that can dissolve or suspend the monomers and that is an organic solvent which does not comprise water. For example, in addition to alcohol type solvents such as methanol, ethanol, propyl alcohol, isopropyl alcohol or butyl alcohol; hydrocarbon type solvents such as hexane, heptane, octane, isooctane, decane or liquid paraffin; ether type solvents such as dimethyl ether, diethyl ether or tetrahydrofuran; ketone type solvents such as acetone or methyl ethyl ketone; ester type solvents such as methyl acetate, ethyl acetate or butyl acetate; and chloride type solvents such as methylene chloride, chloroform or carbon tetrachloride, dimethylformamide, diethylformamide, dimethylsulfoxide, dioxane or the like can be used. These solvents may be used by mixing two or more of them. In usual, it is preferable to select a solvent having higher boiling point than the starting temperature of polymerization initiator used.

As the polymerization initiator, it is not limited in particular as long as it has the ability to start radical polymerization. For example, in addition to peroxides such as benzoyl peroxide and azo compounds such as azobisisobutyronitrile or dimethyl 2,2'-azobis(isobutyrate), persulfuric acid type polymerization initiators such as potassium persulfate or ammonium persulfate can be used. Also, the polymerization may be effected with photochemical reaction or radioactive emission even without depending on these polymerization initiators.

The polymerization temperature is higher than the starting temperature of each polymerization initiator. For example, in case of peroxide type polymerization initiator, the polymerization temperature may be about 70°C in general.

Although the polymerization time is not restricted in particular, it is 2 to 24 hours in usual. It is desirable to react about 1 day in the case where a polymer having relative high molecule weight is expected to obtain. When the reaction time is too short, unreacted monomer will be left and molecular weight will become relative small.

The average molecular weight of the copolymer in accordance with the present invention is not restricted in particular, and it can exhibit the expected effect as long as the copolymer has higher polymerization degree than that of an oligomer. However, as will be mentioned later, cross-linking reaction rate will become slow when polymerization degree is low. Also, applicability or workability will be inferior since viscosity becomes higher when polymerization degree is too large. Accordingly, the average molecular weight of the copolymer is preferably about 2,000 to 150,000.

The copolymer of the present invention comprises the functional silyl groups in its molecule. Therefore, molecules of the copolymer are cross-linked by hydrolyzing, thereby the cross-linked copolymer can be formed. When such cross-linking reaction is conducted on the material to be treated, a coating of the cross-linked copolymer is strongly formed on the surface of the applied portion.

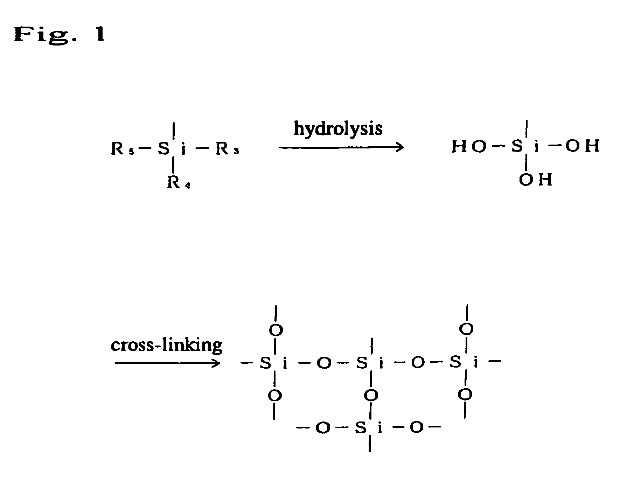

The forming reaction of such cross-linked coat will be considered as follows. Fig. 1 typically shows the reaction comprising monomer (A) as a constitutive monomer. The trifunctional silyl group -SiR₃R₄R₅ of monomer (A) is readily hydrolyzed with water, acid, alkali or the like, thereby turning into a trihydroxysilyl group -Si(OH)₃. This trihydroxysilyl group forms a stable siloxane bond Si-O-Si by reacting with another trihydroxysilyl group. As a result, the copolymer cross-linked (this may refers to the cross-linked copolymer in the present invention) wherein molecules of the copolymer are cross-linked in the form of three-dimensional network, is formed.

Accordingly, when the copolymer is hydrolyzed on the material to be treated, cross-linking occurs on its applied portion, thereby the cross-linked copolymer can be strongly coated on the surface of the material to be treated in network state. However, when the cross-linked copolymer synthesized in advance, is tried to apply on the material to be treated without cross-linking reaction on the material to be treated, it is very difficult to apply such cross-linked copolymer because the cross-linked copolymer becomes a gel or plastic.

A method for forming the cross-linked coat with the copolymer of the present invention is characterized in that a composition of the present invention is hydrolyzed on the material to be treated to cross-link molecules of the copolymer. In the present invention, human hair or human epidermis such as skin, nail, eyelashes, eyebrow or the like is preferable as the material to be treated. Also, materials such as fiber, paper or glass can be treated.

As a method for cross-linking, a reaction with water, acid, or alkali or a reaction with heat can be used. Specifically, after applying the composition of the present invention on the material to be treated, the composition is hydrolyzed to cross-link by bringing the applied portion into contact with water (water vapor or the like may be accepted), acid or alkali or by heating the applied portion. Also, hydrolysis and cross-linking may be conducted by applying the composition on the material treated with water, acid or alkali in advance. Also, the method to apply immediately after water, acid or alkali is mixed with the composition of the present invention may be considered. Generally, however, it is preferable to apply the composition and water, acid, alkali or the like separately. In the case where the composition of the present invention is used as a cosmetic pack, the method to apply immediately after water, acid or alkali is mixed with the composition of the present invention is preferable in terms of strippability. The reaction with water, acid or alkali may be effected with heating. However, it is sufficient to treat at room temperature in usual. Also, it is possible to cross-link naturally with moisture in the atmosphere without bringing the applied portion into contact with acid, water or alkali, when the advance of cross-linking reaction may be slow moving. In any case, instruments such as a brush, a comb, a hair brush or a spray can be used properly as the occasion demands so as to apply and treat the composition uniformly.

An acid and alkali used in the coat-forming method of the present invention are not limited in particular, as long as they can cross-link the copolymers by hydrolysis. An organic or inorganic acid and alkali can be used therefor. One or more of these acids and alkalis may be used, and also the mixture thereof with water may be used.

The composition in accordance with the present invention is characterized by comprising the above-mentioned copolymer as an indispensable ingredient. A preferable embodiment of the composition can be a non-aqueous composition containing said copolymer.

The non-aqueous composition can be, for example, a composition wherein the copolymer is dissolved or dispersed in an organic solvent. Examples of the organic solvent include aliphatic hydrocarbons, aromatic hydrocarbons, chlorine compound type hydrocarbons, ether type solvents, alcohol type solvents, aliphatic mono- to tetra-valent alcohols having 1 to 4 carbon atoms, cellosolve type solvents such as ethylcellosolve or butylcellosolve, dioxane, methyl acetate or dimethylformamide. Among these solvents, aliphatic mono- to di-valent alcohols are preferable as cosmetic ingredients. Examples thereof include methanol, ethanol, isopropanol and propylene glycol. In particular, ethanol or isopropanol is preferable in terms of safety.

In the case where the composition of the present invention is a moisture containing composition, it is preferable to prepare the composition wherein it is applied because the cross-linking reaction will occur in the product. Such moisture containing composition also belongs to the category of the present invention.

A preparation form of the composition in accordance with the present invention is not limited in particular, and any form can be used as long as the effect of the present invention can be exhibited. For example, it can be liquid lotion, milky lotion, cream, gel, mist, spray, aerosol, mousse and the like.

The other ingredients can be comprised into the composition of the present invention within the range that the effect of the present invention is not spoiled. For example, ingredients comprised in cosmetics normally such as surfactants, humectants, ultraviolet protectors, pH adjustors, antiseptics, antioxidants, chelating agents, thickening agents, film-forming ingredients, oily components, polymers or propellants can be used. It is preferable that water, acid or alkali is comprised in an another composition excluding the copolymer. However, it is possible to comprise water, acid or alkali into the composition containing the copolymer of the present invention as mentioned in the above, as long as they are applied to the material to be treated immediately after its preparation.

In the case where other film-forming ingredients are added to the composition along with the copolymer of the present invention, a preferable film-forming ingredient can be the siliconized polysaccharide shown by Formula (XI): 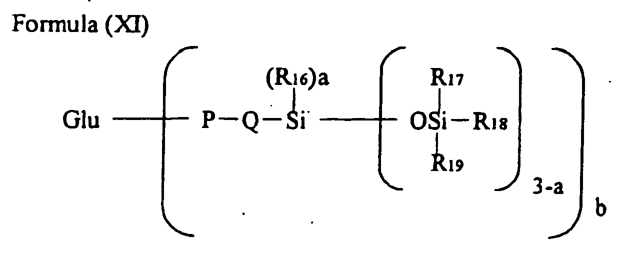 wherein Glu is a sugar residue of a polysaccharide; P is a divalent group for bonding; Q is a divalent aliphatic group; R₁₆ is a monovalent organic group having 1-8 carbon atoms; and R₁₇, R₁₈, and R₁₉ each is a monovalent organic group having 1-8 carbon atoms or a siloxy group shown by -OSiR₂₀R₂₁R₂₂, wherein R₂₀, R₂₁ and R₂₂ each is a monovalent organic group having 1-8 carbon atoms; and a is 0, 1 or 2, and b is an integer of positive number.

In Formula (XI), Glu is a sugar residue of a polysaccharide. As for such polysaccharide, known various polysaccharides can be used. For example, in addition to cellulose, hemicellulose, gum arabic, tragacanth gum, tamarind gum, pectin, starch, mannan, guar gum, locust bean gum, quince seed gum, alginic acid, carrageenan, agar, xanthan gum, dextran, pullulan, chitin, chitosan, hyaluronic acid and chondroitin sulfate, a derivative of these polysaccharides such as polysaccharide derivatives that is effected by carboxymethylation, sulfation, phosphorylation, methylation, ethylation, addition of an alkylene oxide such as ethylene oxide or propylene oxide, acylation, cationization, or lowering of molecular weight can be used. Among them, ethylcellulose or pullulan is preferable and pullulan is more preferable. In the present invention, though an average molecular weight of the polysaccharide is different according to the kind of the polysaccharide, it is preferably about 1,000 to 5,000,000 in general.

These polysaccharides have at least one of reactive functional groups such as a hydroxyl or carboxyl group according to the kinds thereof. The divalent group for bonding shown by P is a group derived from A, which is formed by reacting the reactive functional group of the polysaccharide with a silicone compound shown by the following Formula (XII): 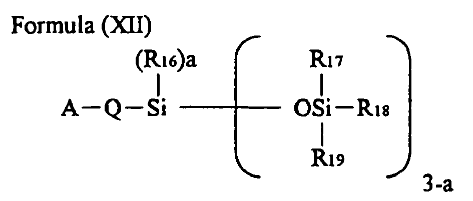 wherein Q, R₁₆, R₁₇, R₁₈, R₁₉ and a are the same as defined in Formula (XI), and A is a functional group that can react with a reactive functional group of a polysaccharide. For example, isocyanate, epoxy, vinyl, acryloyl, methacryloyl, amino, imino, hydroxyl, carboxyl, mercapto group or the like can be used as A.

In the reaction between such silicone compound and polysaccharide, known methods such as the method described in Japanese Unexamined Patent Publication No. 8-134103, can be used.

Example of P include carbamoyl group, -CH₂CH(OH)-, carbonyl group, amino group and ether group. Among them, a carbamoyl group -CONH-, which is formed by reaction of the compound of Formula (XII) wherein A is isocyanate group (O=C N-) with a hydroxyl group of the polysaccharide, is preferable. The sugar residue of the polysaccharide in this case represents the rest of the polysaccharide excluding the hydrogen atom of the hydroxyl group reacting with the isocyanate group. Also, in the other reaction, the definition of a sugar residue of a polysaccharide is according thereto.

As for the divalent aliphatic group shown by Q, alkylene group, alkylene group comprising oxygen atom, nitrogen atom, sulfur atom or the like in main chain, alkylene group comprising an arylene group such as phenylene group in main chain, and alkylene group comprising carbonyloxy group or oxycarbonyl group in main chain can be used. These divalent aliphatic groups can have a substituent such as hydroxy, alkoxy, alkyl group or the like, and the terminal atom of the aliphatic group may be a hetero atom such as oxygen atom, nitrogen atom or sulfur atom. Q can be -(CH₂)₂-, -(CH₂)₃-,-(CH₂)₄-, -(CH₂)₆-, -(CH₂)₈-, -{CH₂CH(CH₃)}-, -(CH₂)₂O(CH₂)₃-,-CH₂CH(OH)-CH₂- or the like. Propylene group shown by -(CH₂)₃- is preferable.

In Formula (XI), the monovalent organic group having 1-8 carbon atoms shown in R₁₆, R₁₇, R₁₈, R₁₉, R₂₀, R₂₁ and R₂₂, can be an alkyl group such as methyl, ethyl, propyl or butyl group; a cycloalkyl group such as cyclopentyl or cyclohexyl group; an aryl group such as phenyl group; an aralkyl group such as benzyl group; an alkenyl group such as vinyl or allyl group, an fluorinated alkyl group such as 3,3,3-trifluoropropyl group; or the like can be listed. Alkyl group is preferable as such organic group, and methyl group is more preferable.

Also, each of R₁₇, R₁₈ and R₁₉ may be a siloxy group shown by -OSiR20R21R22. Examples of the siloxy group include a trimethylsiloxy, ethyldimethylsiloxy, phenyldimethylsiloxy, vinyldimethylsiloxy and 3,3,3-trifluoropropyldimethylsiloxy group. Also, R₁₆, R₁₇, R₁₈, R₁₉, R₂₀, R₂₁ and R₂₂ may be the same or different each other. In the siliconized polysaccharide of the present invention, it is preferable that a=0 and R₁₇, R₁₈ and R₁₉ each is methyl group.

In the present invention, more preferable siliconized polysaccharide may be a siliconized pullulan shown by the following Formula (XIII): 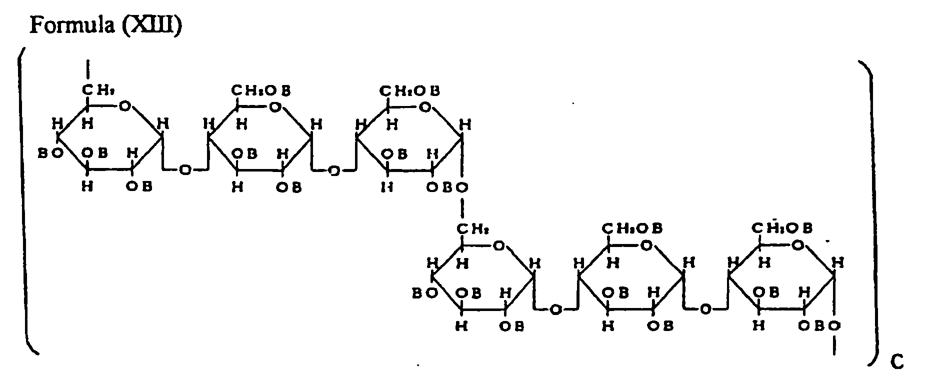 wherein B is a hydrogen atom or a group shown by -CONH(CH₂)₃Si[OSi(CH₃)₃] and its substitution degree is 0.5-2.5; and c is a number from 100 to 20,000.

In the present invention, the substitution degree of the siliconized polysaccharide represents an average number of the silicone compound bonding to one unit of constituent sugar of the polysaccharide. For example, the substitution degree of the above-mentioned siliconized pullulan indicates the average number of the substituent - CONH(CH₂)₃Si[OSi(CH₃)₃] bonding to the base unit of pullulan shown by the following Formula (XIV):

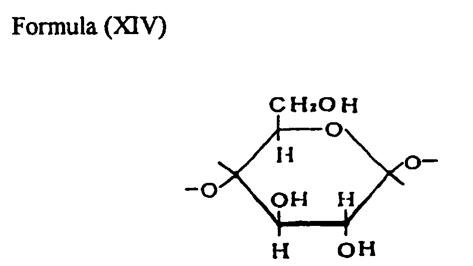

Hair nature modifying effect, damaged hair recovering effect, resistance to shampoo, coloring retention effect or the like can be improved by using the copolymer containing reactive silyl groups together with the siliconized polysaccharide. Further, flaking at the time when the composition is repeatedly applied can be inhibited.

In the case of compounding the siliconized polysaccharide, when the siliconized polysaccharide dissolving into a solvent such as a silicone oil having low molecular weight or light isoparaffin is used, facility for compounding and feeling of use at the application time can be ameliorated. As for the silicone oil having low molecular weight, linear dimethyl polysiloxane having 2-7 carbon atoms or cyclic dimethyl polysiloxane having 3-7 carbon atoms is preferable. Although an amount of the siliconized polysaccharide in the composition is not restricted in particular, it is 0.01 to 20 wt% in general and, preferably, 0.2 to 10wt%.

In the following, the effect of the composition in accordance with the present invention will be described in detail. Structures of each monomer used in the following are shown below. Also, the compounding amount is wt% unless otherwise stated.

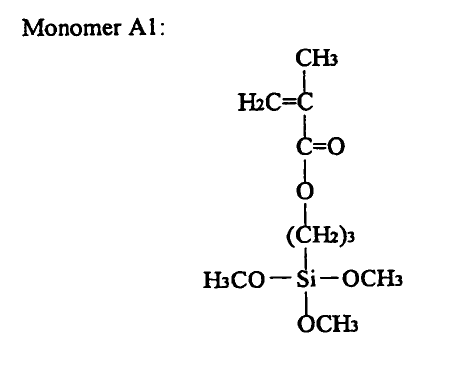

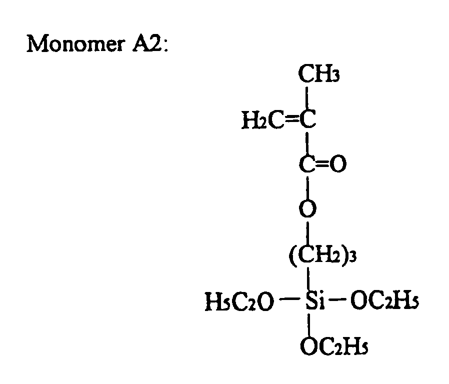

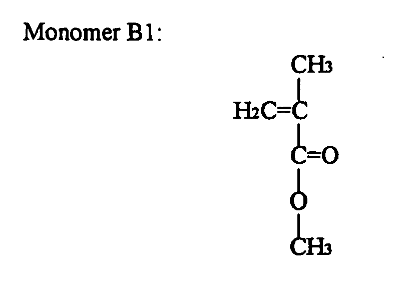

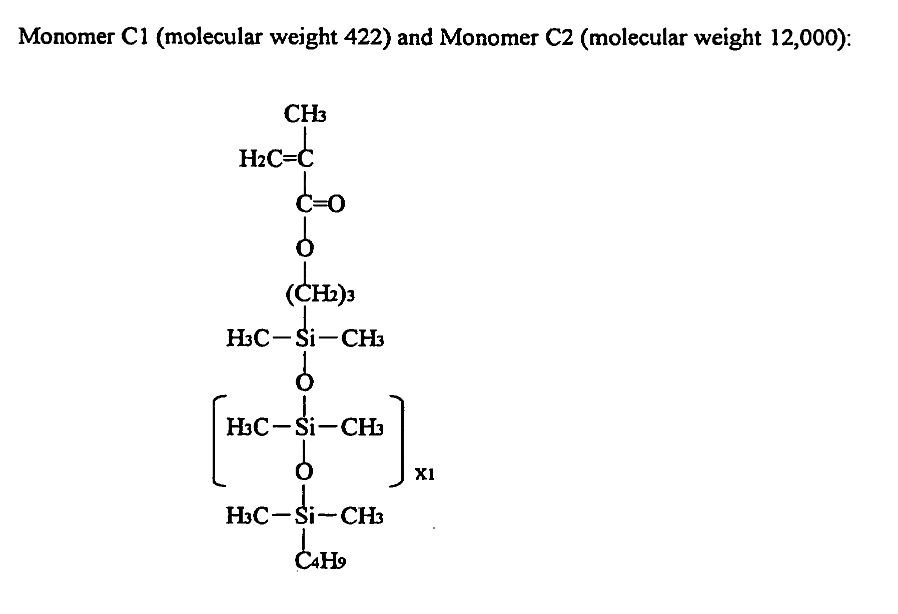

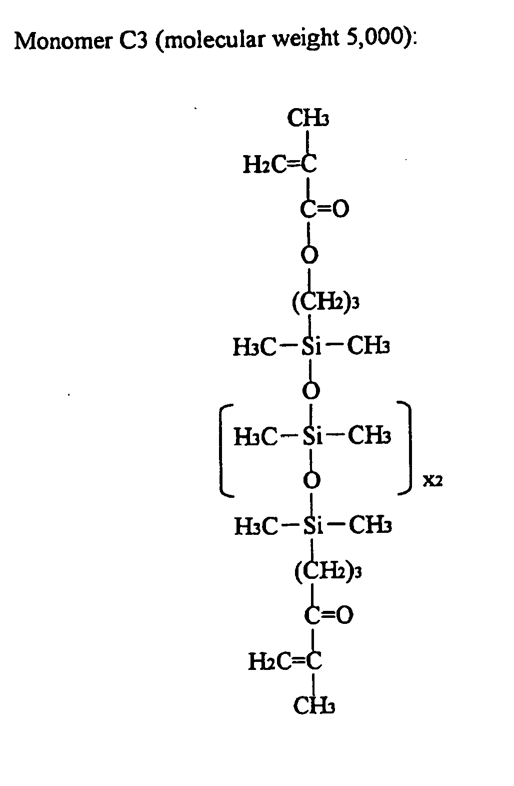

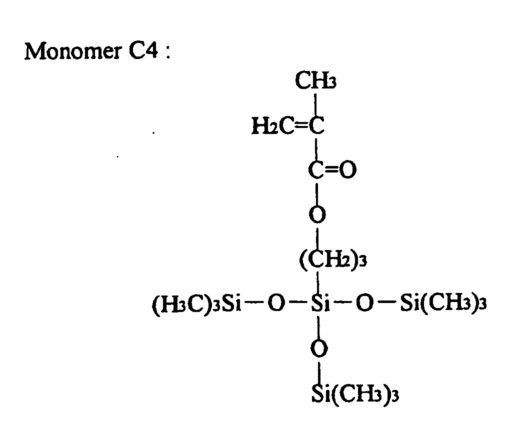

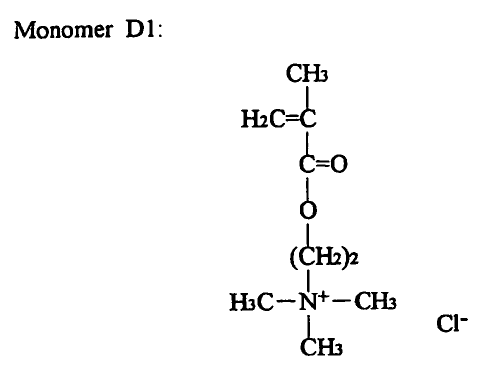

### < Cosmetics for Hair Coating >

A lot of people are not satisfied with respect to their own hairs. For example, the people who have soft hair desire to give tension and hardness to their hair. A lot of hair treatment preparations and hair styling preparations has been developed.

Also, irrespective of age or sex, coloring of hair according to their own taste has been conducted in usual so as to get rid of discontent concerning the colors of their own hair. This can be considered that the spread of temporary hair colors such as color spray and semipermanent hair color such as acidic hair color that can easily color hair without damaging, largely influenced thereto.

However, any of such conventional hair treatment or hair styling preparations are effective in nothing but temporary. Because swimming, bathing or shampoo washes off them, the effects are lost. Accordingly, as the nature of hair can not be improved fundamentally even when such preparations are used, it is necessary to continuously use such hair cosmetic preparations every day. Further, there is very few hair cosmetic preparations that can exhibit satisfactory effect.

Also, as the color of hair by temporary hair color or acidic hair color is easily washed off by shampoo or the like, it is desired to maintain its coloring retention.

Therefore, the effect when the composition comprising a copolymer of the present invention was treated on hair, was studied.

### Test Example 1-1

### i) Synthesis of Copolymer

### Copolymer 1-1

2.Og (8mmol) of 3-methacryloxypropyltrimethoxysilane (Monomer A1) and 8.0g (80mmol) of methyl methacrylate (Monomer B1) were dissolved into 100ml of ethanol and the mixture was heated with stirring under nitrogen gas stream for 1 hour at 70°C. 0.05g of potassium persulfate was added to the solution and the mixture was reacted over night. The reaction mixture was cooled down to room temperature and concentrated under a vacuum. The residue was dissolved into 10ml of ethanol and the solution was added to 500ml of n-hexane. The deposits were collected to obtain the aimed copolymer.

In the NMR spectral data (solvent CDCl₃ or DMSO - d₆) of the resulting copolymer, a peak of hydrogen atom of CH₂=C derived from a monomer used as a starting material, that the peak should be observed at 6-7 ppm or near, was not observed. As a result, generation of copolymer was confirmed. Also, in the present invention, generation of the copolymers was confirmed in the similar manner.

### Copolymer 1-2

In the similar manner to Copolymer 1-1, except for using 4.0g (16mmol) of 3-methacryloxypropyltrimethoxysilane and 8.0g (80mmol) of methyl methacrylate, the aimed copolymer was obtained.

### Copolymer 1-3

In the similar manner to Copolymer 1-1, except for using 8.0g (32mmol) of 3-methacryloxypropyltrimethoxysilane and 4.0g (40mmol) of methyl methacrylate, the aimed copolymer was obtained.

### Copolymer 1-4

In the similar manner to Copolymer 1-1, except for using 10.0g (40mmol) of 3-methacryloxypropyltrimethoxysilane and 2.0g (20mmol) of methyl methacrylate, the aimed copolymer was obtained.

### Copolymer 1-5

In the similar manner to Copolymer 1-1, except for using 10.0g (40mmol) of 3-methacryloxypropyltrimethoxysilane and 0.4g (4mmol) of methyl methacrylate, the aimed copolymer was obtained.

### Copolymer 1-6

In the similar manner to Copolymer 1-1, except for using 10.0g (40mmol) of 3-methacryloxypropyltrimethoxysilane, the aimed copolymer was obtained.

### ii) Test Method

Each Copolymers 1-1 to 1-6 was tested by the following method. 3 parts by weight of each copolymer was dissolved into 97 parts by weight of ethanol and the solution which was filled into a spray container was used as a sample.

### (Hair nature modifying effect)

20 of professional panel were used for evaluating with feelings. Approximately 2g of the spray solution was sprayed on hairs of each panel and the hair was had with a commercially available shampoo. The tension and hardness after drying hair naturally were asked by means of questionnaires and was evaluated by the following standard.
⊚: Among 20 panel, 16 or more answered their own hair had tension and hardness.
O : Among 20 panel, 12 to 15 answered their own hair had tension and hardness.
Δ: Among 20 panel, 6 to 11 answered their own hair had tension and hardness.
× : Among 20 panel, 0 to 5 answered their own hair had tension and hardness.

### (Resistance to shampoo)

After studying hair nature modifying effect, hair washing with shampoo and drying each was repeated in 3 cycles. The tension and hardness were evaluated with feelings similarly. Evaluation standard was as follows.
⊚: Among 20 panel, 16 or more answered that the effect was maintained and resistance to shampoo was exhibited.
O : Among 20 panel, 12 to 15 answered that the effect was maintained and resistance to shampoo was exhibited.
Δ : Among 20 panel, 6 to 11 answered that the effect was maintained and resistance to shampoo was exhibited.
× : Among 20 panel, 0 to 5 answered that the effect was maintained and resistance to shampoo was exhibited.

### (Flaking)

In hair nature modifying effect test mentioned above, presence of flaking was judged with visual inspection. Evaluation standard was as follows.
⊚ : Flaking was not observed absolutely.
Δ : Flaking was observed slightly.
× : Flaking was observed clearly.

**TABLE 1**

| | Monomer (wt%) | Hair nature | Resistance | Flaking |
|---|---|---|---|---|
| Copolymer | A1/B1 | modifying effect | to shampoo | |
| 1-1* | 20/80 | ⊚ | Δ | ⊚ |
| 1-2* | 33/67 | ⊚ | ○ | ⊚ |
| 1-3* | 67/33 | ⊚ | ⊚ | ⊚ |
| 1-4* | 83/17 | ⊚ | ⊚ | ⊚ |
| 1-5* | 96/4 | ○ | ○ | Δ |
| 1-6* | 100/0 | Δ | Δ | Δ |

| | | | | |
|---|---|---|---|---|
| (*) does not fall within the scope of the present invention | | | | |

From TABLE 1, it is understood that a coating of the cross-linked copolymer containing Monomer A as a constitutive monomer possesses hair nature modifying effect.

Because resistance to shampoo was not obtained sufficiently, and it was inferior in continuity of hair nature modifying effect in the case where Monomer (A) was too less in the copolymer. On the other hand, both hair nature modifying effect and resistance to shampoo were tended to be lowered and flaking was generated easily, in the case where Monomer (A) was 100%. Therefore, as for monomer composition of the copolymer, Monomer (A) in the copolymer is 25 to 99 wt% and preferably is 40 to 85 wt%.

### Test Example 1-2

The spray prepared in Test Example 1-1 was used as a sample and test for coloring retention was conducted by the following method.

### (Coloring retention)

A commercially available acidic hair color was uniformly applied on a bunch of white human hair. 3 minutes later, the bunch was washed with water and dried naturally. 5g of the colored human hair bunch (control) was taken and approximately 1g of the sample was sprayed thereon. Hair washing with commercially available shampoo and natural drying were repeated ten times with respect to the human hair bunch with or without treatment of the spray. Change of color of thus shampoo-treated human hair bunch with respect to the control that was not treated with shampoo was observed with naked eyes and evaluated.
⊚ : Absolutely no discoloring was occurred.
O : Almost no discoloring was occurred.
Δ : Discoloring was occurred.
× : Discoloring was occurred remarkably.

**TABLE 2**

| | Monomer (wt%) | Coloring |
|---|---|---|
| Copolymer | A1/B1 | retention |
| 1-1* | 20/80 | Δ |
| 1-2* | 33/67 | ○ |
| 1-3* | 67/33 | ⊚ |
| 1-4* | 83/17 | ⊚ |
| 1-5* | 96/4 | ○ |
| 1-6* | 100/0 | Δ |
| Nothing | - | × |
| (not sprayed) | | |

| | | |
|---|---|---|
| (*) does not fall within the scope of the present invention | | |

As was understood from TABLE 2, discoloring caused by shampoo was remarkably observed in the case where the hair was not treated with the copolymer. However, in the hair treated with the copolymer, discoloring was inhibited and coloring retention was improved. Therefore, a coat of the cross-linked copolymer in accordance with the present invention possesses coloring retention improving effect when the copolymer was treated on the colored hair. It is understood that the effect is remarkably exhibited in the case where the percentage of Monomer (A) in the copolymer is 25 to 99 wt% and, preferably, 40 to 85 wt%.

### Test Example 1-3

Next, the copolymers that has monomer composition of TABLE 3 were synthesized in the same way as Test Example 1-1 and tested similar to Test Examples 1-1 and 1-2. Also, smoothness and texture were evaluated according to the following method.

### (Smoothness)

Smoothness of the hair after hair nature modifying effect test was asked by means of questionnaires and was evaluated according to the following standard.
⊚ : Among 20 panel, 16 or more answered that feeling of use was smooth.
O : Among 20 panel, 12 to 15 answered that feeling of use was smooth.
Δ : Among 20 panel, 6 to 11 answered that feeling of use was smooth.
× : Among 20 panel, 0 to 5 answered that feeling of use was smooth.

### (Texture)

Texture of the hair after hair nature modifying effect test was asked by means of questionnaires and was evaluated according to the following standard.
⊚ : Among 20 panel, 15 or more answered favorable.
○ : Among 20 panel, 10 to 14 answered favorable.
Δ : Among 20 panel, 5 to 9 answered favorable.
× : Among 20 panel, 0 to 5 answered favorable.

**TABLE 3**

| | Monomer(wt%) | | | Hair nature | Resistance | Flaking | Coloring | Smoothness |
|---|---|---|---|---|---|---|---|---|
| Copolymer | A1 | B1 | C* | modifying effect to shampoo | | | retention | |
| 1-7⁺ | 33 | 67 | - | ⊚ | ○ | ⊚ | ○ | Δ |
| 1-8⁺ | 67 | 33 | - | ⊚ | ⊚ | ⊚ | ⊚ | Δ |
| 1-9⁺ | 50 | 10 | 40(C1) | ⊚ | ⊚ | ⊚ | ⊚ | ⊚ |
| 1-10⁺ | 50 | 10 | 40(C2) | ⊚ | ⊚ | ⊚ | ⊚ | ⊚ |
| 1-11⁺ | 50 | 40 | 10(C3) | ⊚ | ⊚ | ⊚ | ⊚ | ⊚ |
| -12⁺ | 60 | 35 | 5(C4) | ⊚ | ⊚ | ⊚ | ⊚ | ⊚ |
| 1-13⁺ | 50 | - | 50(C2) | Δ | ⊚ | ⊚ | ⊚ | ⊚ |
| 1-14⁺ | 25 | - | 75(C4) | Δ | ○ | ⊚ | ○ | ⊚ |

| | | | | | | | | |
|---|---|---|---|---|---|---|---|---|
| * The inside of ( ) represents Monomer (C) used. (+) does not fall within the scope of the present invention | | | | | | | | |

As is understood from TABLE 3, hair nature modifying effect, resistance to shampoo and coloring retention are improved and feeling of use in hair after treatment can be remarkably smoothed by introducing Monomer (C) into the copolymer consisted of Monomers (A) and (B). Also, although hair nature modifying effect was low and the copolymer was difficult to treat due to stickiness in the copolymers 1-13 and 1-14, these copolymers had well property in resistance to shampoo, coloring retention effect and smoothness.

As shown in the foregoing, the percentage of Monomer (C) in the copolymer is 1 wt% or more and, preferably, 5 wt% or more. It was also indicated that Monomer (B) in the copolymer contributed to hair nature modifying effect-and prevention of stickiness of the copolymer and that the percentage of Monomer (B) in the copolymer was 1 wt% or more and, preferably, 10 wt% or more.

**TABLE 4**

| | Monomer(wt%) | | | | Hair nature | Resistance | Coloring | Texture |
|---|---|---|---|---|---|---|---|---|
| Copolymer | A2 | B1 | C4 | D1 | modifying effect | to shampoo | retention | |
| 1-12* | 60 | 35 | 5 | - | ⊚ | ⊚ | ⊚ | Δ |
| 1-15 | 60 | 35 | 5 | 10 | ⊚ | ⊚ | ⊚ | ⊚ |
| 1-16 | 60 | 35 | 5 | 20 | ⊚ | ○ | ○ | ⊚ |
| 1-17 | 60 | 35 | 5 | 50 | ○ | ○ | ○ | ⊚ |
| 1-18 | 60 | 35 | 5 | 100 | Δ | × | × | ⊚ |

| | | | | | | | | |
|---|---|---|---|---|---|---|---|---|
| (*) does not fall within the scope of the present invention | | | | | | | | |

As is understood from TABLE 4, the copolymer consisted of Monomers (A) to (D) can give smoothness, good texture and moist feeling to hair. In the case where the percentage of Monomer (D) was high, hair nature modifying effect, resistance to shampoo and coloring retention tended to be lowered because the hydrophilic nature of the cross-linked coat becomes high. Accordingly, the percentage of Monomer (D) is 1 to 100 wt% and, more preferably, 5 to 50 wt% with respect to the whole amount of Monomers (A) to (C) in the copolymer.

As mentioned above, by effecting cross-linking reaction after applying the composition comprising the copolymer having reactive silyl groups on hair, tension and hardness are given to the hair. At the same time, an excellent resistance to shampoo can be exhibited, thereby the effect is maintained without loss even after repeating shampoo. Also, feeling of use is not stiffened and flaking does not occurred. Further, in the case where this treatment was effected on colored hair, discoloring at the time of shampoo is inhibited and coloring retention is remarkably improved.

It can be considered that because cross-linking reaction occur on hair by hydrolyzing the copolymer and the cross-linked coat, which is excellent in resistance to washing, strongly coats the hair in network state, these effects are exhibited.

Accordingly, the composition of the present invention is useful as a cosmetic for hair coating or for modifying the nature of hair.

A preferable example of the copolymer to be comprised into the cosmetic for hair coating or for modifying the nature of hair in accordance with the present invention can be shown by Formula (X).

As a monomer composition of the copolymer, the percentage of Monomer (A) in the copolymer is 25 to 99 wt% and, more preferably, 40 to 85 wt%. In the case where Monomer (A) is too little, resistance to shampoo is low and hair nature modifying effect and coloring retention effect may not be sufficiently maintained since the part for cross-linking is few. On the contrary, hair nature modifying effect, coloring retention and resistance to shampoo tend to be lowered in the case where Monomer (A) is too much. Also, flaking is easy to occur. These can be considered that cross-linking reaction is not progressed neatly and a large amount of the unreacted part is remained in the copolymer because the part for cross-linking is too much to be in close state.

Monomer (B) adjusts the ratio of Monomer (A) and contributes to hair nature modifying effect and water-repellency of the coat of the cross-linked copolymer. Monomer (B) also inhibits flaking. The percentage of Monomer (B) is 1 wt% or more and, more preferably, 10 wt% or more in the copolymer. The percentages of the other monomers are decreased and the solubility of the copolymer will become poor against alcohol type solvents in the case where Monomer (B) is too much. Therefore, the percentage of Monomer (B) is 75 wt% or less and, preferably, 60 wt% or less.

Monomer (C) has a siloxane part to improve water repellency and resistance to shampoo of the cross-linked coat and to give the hair after treatment smoothness in feeling of use. Monomer (C) can also inhibits flaking. The percentage of Monomer (C) is 1 wt% or more and, preferably, 5 wt% or more in the copolymer of the present invention. However, when the percentage of Monomer (C) is too large, the percentages of the other monomers become relatively small and resistance to shampoo tends to be poor. Therefore, the percentage of Monomer (C) is 70 wt% or less and, preferably, 60 wt% or less.

Also, when the copolymer comprising Monomer (D) as a constitutive monomer is treated, good texture can be given to hair and moist feeling of use can be obtained. However; when the percentage of Monomer (D) is too large, water-repellence, resistance to shampoo and the like become poor because hydrophilic nature of the coat becomes high due to amine portion contained in Monomer (D). The percentage of Monomer (D) is 1 to 100 wt% and, preferably, 5 to 50 wt% with respect to a whole amount of Monomers (A) to (C).

Although a concentration of the copolymer is not restricted in particular, it is preferably 0.1 to 10 wt% and, more preferably, 1 to 5 wt%. When an amount of the copolymer is too little, the sufficient effect can not be obtained at one time treatment. On the contrary, when the amount of the copolymer is too much, applicability, spreadability, feeling of use and the like become inferior and flaking may occur.

The present inventors further studied about the effect at the time when the copolymer of the present invention and the other film-forming ingredients were used together. As for the film-forming ingredient, siliconized pullulan (substitution degree is 1.7, molecular weight of pullulan used as a starting material is approximately 200,000), which is one of siliconized polysaccharide was used.

### Test Example 1-4

A sample was prepared according to the prescription of TABLE 5 and tested by the following method.

### (Hair nature modifying effect)

20 of professional panel evaluated with feelings. After approximately 2g of the sample was sprayed on hair of each panel, the hair was washed with water. The question concerned with the tension and hardness of hair after drying naturally were asked by means of questionnaires and was evaluated by the following standard (just after the test). Also, the tension and hardness of hair after repeatedly washing with a commercially available shampoo and natural drying 5 times were also evaluated by the following standard.
⊚ : 16 or more answered their own hair had tension and hardness.
○ : 12 to 15 answered their own hair had tension and hardness.
Δ : 6 to 11 answered their own hair had tension and hardness.
× : 0 to 5 answered their own hair had tension and hardness.

### (Damaged hair recovering effect)

20 of professional panel who had damaged hair were evaluated with feelings. Approximately 2g of the sample was sprayed on hair of each panel and then the hair was washed with water. The question concerned with the touch (smoothness) after natural drying was asked by means of questionnaires and was evaluated by the following standard (just after the test). Also, the touch after repeatedly washing with commercially available shampoo and natural drying 5 times was also evaluated by the following standard.
⊚ : 16 or more answered that the touch was smooth and damaged hair recovering effect was obtained.
○ : 12 to 15 answered that the touch was smooth and damaged hair recovering effect was obtained.
Δ : 6 to 11 answered that the touch was smooth and damaged hair recovering effect was obtained.
× : 0 to 5 answered that the touch was smooth and damaged hair recovering effect was obtained.

### (Coloring retention)

Coloring retention had tested according to the method of Test Example 1-2.

### (Flaking on repeated application)

After approximately 2g of the sample was sprayed on the hair of 20 professional panel each, the hair was washed with water and dried naturally. Presence of flaking after repeating the above-mentioned cycle 3 times was judged with visual inspection. Evaluation standard is as follows.
⊚ : Flaking was not observed at all.
Δ : Flaking was observed slightly.
× : Flaking was observed clearly.

**TABLE 5**

| Ingredient* | Amount | | | | | | | | | | |
|---|---|---|---|---|---|---|---|---|---|---|---|
| Copolymer 1-19 | 5 | - | - | - | - | - | 5 | - | - | - | - |
| Copolymer 1-20⁺ | - | 5 | - | - | - | - | - | 5 | - | - | - |
| Copolymer 1-21⁺ | - | - | 5 | - | - | - | - | - | 5 | - | - |
| Copolymer 1-22⁺ | - | - | - | 5 | - | - | - | - | - | 5 | - |
| Copolymer 1-23⁺ | - | - | - | - | 5 | - | - | - | - | - | 5 |
| Siliconized pullulan | - | - | - | - | - | 5 | 0.5 | 0.5 | 0.5 | 0.5 | 0.5 |
| Ethanol | 35 | 35 | 35 | 35 | 35 | 40 | 35 | 35 | 35 | 35 | 35 |
| Light isoparaffin | 60 | 60 | 60 | 60 | 60 | 55 | 59.5 | 59.5 | 59.5 | 59.5 | 59.5 |

| Hair nature modifying effect | | | | | | | | | | | |
|---|---|---|---|---|---|---|---|---|---|---|---|
| Just after test | ⊚ | ⊚ | ⊚ | ⊚ | Δ | Δ | ⊚ | ⊚ | ⊚ | ⊚ | ○ |
| After 5 times | ○ | ⊚ | ⊚ | Δ | Δ | ⊚ | ⊚ | ⊚ | ⊚ | ⊚ | ○ |
| shampoo | | | | | | | | | | | |

| Damaged hair recovering effect | | | | | | | | | | | |
|---|---|---|---|---|---|---|---|---|---|---|---|
| Just after test | ○ | ○ | ○ | Δ | ○ | ⊚ | ⊚ | ⊚ | ⊚ | ⊚ | ⊚ |
| After 5 times | Δ | ○ | ○ | Δ | ○ | ⊚ | ⊚ | ⊚ | ⊚ | ⊚ | ⊚ |
| shampoo | | | | | | | | | | | |
| Coloring retention | ○ | ⊚ | ⊚ | ⊚ | ⊚ | Δ | ⊚ | ⊚ | ⊚ | ⊚ | ⊚ |
| Flaking on | Δ | Δ | Δ | × | Δ | ⊚ | ⊚ | ⊚ | ⊚ | ⊚ | ⊚ |
| repeated application | | | | | | | | | | | |

| | | | | | | | | | | | |
|---|---|---|---|---|---|---|---|---|---|---|---|
| * The monomers (wt%) of each copolymer are as follows. Copolymer 1-19 : A2-BI-C3-D1=60-35-5-20 Copolymer 1-20⁺: A2-B1-C1=50-40-10 Copolymer 1-21⁺ A2-B1-C2=50-25-25 Copolymer 1-22⁺: A2-B1=67-33 Copolymer 1-23⁺: A2-C3=50-50 (⁺) does not fall within the scope of the present invention | | | | | | | | | | | |

As shown in TABLE 5, when the copolymer and siliconized pullulan were used together, hair nature modifying effect and coloring retention effect can be equal to or be improved as compared with the case where the copolymer only is used. Also, the touch could be more smoothed as compared with the case where the copolymer only was used and the effect was remarkable with respect to damaged hair. Also, these effects were maintained even after repeating shampoo. Further, though flaking by repeated application of the copolymer may be occurred in the case where the copolymer only was used, it can be inhibited by using siliconized pullulan together.

**TABLE 6**

| Ingredient | Amount | | | | | | | |
|---|---|---|---|---|---|---|---|---|
| Copolymer 1-19 | 5 | 5 | 5 | 2 | 2 | 1 | 0.1 | - |
| Siliconized pullulan | - | 0.1 | 0.5 | 0.5 | 2 | 5 | 5 | 5 |
| Ethanol | 35 | 35 | 35 | 38 | 39 | 39 | 39.9 | 40 |
| Light isoparaffin | 60 | 59.9 | 59.5 | 59.5 | 59 | 55 | 55 | 55 |

| Hair nature modifying effect | | | | | | | | |
|---|---|---|---|---|---|---|---|---|
| Just after test | ⊚ | ⊚ | ⊚ | ⊚ | ⊚ | ⊚ | ○ | Δ |
| After 5 times | ○ | ⊚ | ⊚ | ⊚ | ⊚ | ○ | Δ | Δ |
| shampoo | | | | | | | | |

| Damaged hair recovering effect | | | | | | | | |
|---|---|---|---|---|---|---|---|---|
| Just after test | ○ | ○ | ⊚ | ⊚ | ⊚ | ⊚ | ⊚ | ⊚ |
| After 5 times | Δ | ○ | ⊚ | ⊚ | ⊚ | ⊚ | ⊚ | ⊚ |
| shampoo | | | | | | | | |
| Coloring retention | ○ | ⊚ | ⊚ | ⊚ | ⊚ | ○ | Δ | × |
| Flaking on | Δ | ⊚ | ⊚ | ⊚ | ⊚ | ⊚ | ⊚ | ⊚ |
| repeated application | | | | | | | | |

TABLE 6 is the result that the test was conducted as in Test Example 1-1 with changing the ratios of the copolymer and siliconized pullulan. Hair nature modifying effect and coloring retention tends to be lowered in the case where the copolymer is little. On the other hand, hair nature modifying effect, coloring retention and damaged hair recovering effect were not sufficiently improved and flaking on repeated application may occur in the case where siliconized pullulan is little. Accordingly, the ratio between the copolymer and siliconized pullulan by weight is preferably 100 : 1 and, more preferably, 50:1-1:1.

As described in the above, by using a copolymer containing reactive silyl groups together with a siliconized polysaccharide, a composition that hair nature modifying effect, coloring retention and damaged hair recovering effect is high and maintained at the time when shampoo is repeated, and flaking on repeated application is little, can be obtained.

In the case where only the siliconized polysaccharide is used, hair nature modifying effect and coloring retention effect are low as compared with the case where only the copolymer was used. Therefore, it can be considered that these effects are improved by using the siliconized polysaccharide together with, because the siliconized polysaccharide is included into the cross-linked coat, thereby, at the time of forming the cross-linked coat, the cross-linked coat and the siliconized polysaccharide are synergistically contributed.

### Example 1-1 Hair Coat

| | |
|---|---|
| (1) Copolymer 1-19 | 3 wt% |
| (2) Siliconized pullulan | 0.5 |
| (3) Stearyltrimethylammonium chloride | 0.5 |
| (4) Ethanol | 37 |
| (5) Light isoparaffin | 19 |
| (6) LPG (3.5kg/cm² at 20°C) | 40 |
| (7) Perfume | q.s. |

### (Manufacturing Process)

Siliconized pullulan was dissolved into light isoparaffin. The ethanol solution of dimethylpolysiloxane, perfume, copolymer and stearyltrimethylammonium chloride was added thereto. The mixture and LPG were filled into an aerosol container in order, thereby obtaining a hair coat.

This hair coat could improve coloring retention of the hair that was colored with temporary or acidic hair dye, as compared with the hair coat that siliconized pullulan was not compounded thereto.

### < Cosmetics Containing Pigment and/or Acidic Dye >

A pigment or dye is used for various cosmetics as a coloring material.

Among such cosmetics, a hair color is classified broadly into: a temporary hair color which comprises a pigment as a main coloring ingredient and makes the coloring ingredient adhere only on the surface of the hair (e.g., color stick, color spray or the like); a semipermanent hair color (acidic hair color) which comprises an acidic dye as a main coloring ingredient and make a part of the coloring ingredient penetrate into the inside of hair; and a permanent hair color (oxidation hair color) which comprises an oxidation dye color as a main coloring ingredient and colors to deep part of hair.

Among these hair colors, though the oxidation hair color can maintain color thereof, it has problems such as hair damage easily caused, eruption or coloring of scalp, and its difficulty on treatment.

On the contrary, though the temporary and semipermanent hair colors do not cause hair damage and skin irritation and can be treated conveniently, there are disadvantages that color retention is poor and the hair colors are easy to come off. Accordingly, it is desired to improve color retention of such temporary or semipermanent hair color.

In addition to such hair cosmetics, a pigment or dye is an essential ingredient in makeup cosmetics. It is also desired to improve water-repellency and cosmetic retention in such the makeup cosmetics.

Therefore, the effect in the case where the copolymer containing reactive silyl groups was comprised into the cosmetic containing a pigment or acidic dye was studied.

### Test Example 2-1

### i) Preparation of Sample

### Prescription 2-1 :

| | |
|---|---|
| (1) Copolymer | 2 or 3 wt% |
| (2) Carbon black | 2 |
| (3) Ethanol | 44.8 or 43.8 |
| (4) Beeswax | 10 |
| (5) Japanese wax | 10 |
| (6) Castor oil | 30 |
| (7) POE (20) sesquioleate | 1.2 |
| (8) Perfume | q.s. |
| (9) Antioxidant | q.s. |

### (Manufacturing Process)

Oily ingredients and carbon black were added into an ethanol solution of the copolymer, successively. After adding perfume and antioxidant into the mixture, a temporary hair color was obtained by stirring sufficiently.

### ii) Test Method

Each sample was applied on white hair and the hair was left for 3 minutes. Then the hair was washed with water and dried naturally. Presence of flaking was evaluated at this time. Thereafter, the hair was washed with a commercially available shampoo and dried naturally, to evaluate coloring retention by visual inspection. Also, a hair color, which comprises ethanol instead of the copolymer, was prepared as a control and tested in the same manner. Evaluation standard is as follows.

### (Evaluation standard of coloring retention)

⊚ : Coloring effect was maintained for 7 days or more.
○ : Coloring effect was maintained for 4-6 days.
Δ : Coloring effect was maintained for 2-3 days.
× : No durability.

### (Evaluation standard of flaking)

⊚ : Flaking was not observed at all.
Δ : Flaking was observed slightly.
× : Flaking was observed clearly.

**TABLE 17**

| Copolymer | Monomer (wt%).. | Coloring Retention | | Flaking |
|---|---|---|---|---|
| | A1/B1 | 2 wt% | 3 wt% | 3 wt% |
| 2-1⁺ | 20/80 | Δ | ○ | ⊚ |
| 2-2⁺ | 33/67 | ○ | ⊚ | ⊚ |
| 2-3⁺ | 67/33 | ○ | ⊚ | ⊚ |
| 2-4⁺ | 83/17 | ○ | ⊚ | ⊚ |
| 2-5⁺ | 96/4 | ○ | ⊚ | ⊚ |
| 2-6⁺ | 100/0 | Δ | ○ | Δ |
| Nothing | - | × | × | ⊚ |
| (Ethanol) | | | | |

| | | | | |
|---|---|---|---|---|
| (⁺) does not fall within the scope of the present invention | | | | |

As is clear from TABLE 17, it is understood that the temporary hair color which comprises the copolymer is excellent in resistance to washing and is considerably improved in coloring retention as compared with the case that the copolymer is not comprised.

### Test Example 2-2

A liquid acidic hair color was prepared according to the following prescription 2-2 and coloring retention and flaking were tested in the same way as the above-mentioned Test Example 2-1.

### Prescription 2-2:

| | |
|---|---|
| Copolymer | 2 or 3 wt% |
| Acidic dye (Natrozole) | 1 |
| Ethanol | 20:7 or 19.7 |
| Benzyl alcohol | 6 |
| Isopropyl alcohol | 20 |
| Lactic acid | 0.3 |
| Silicone KF-56 (manufactured by Shin-Etsu Chemical Co., Ltd.) | 50 |
| Perfume | q.s. |
| Antioxidant | q.s. |

**TABLE 18**

| Copolymer | Monomer (wt%) | Coloring Retention | | Flaking |
|---|---|---|---|---|
| | A1/B1 | 2 wt% | 3 wt% | 3 wt% |
| 2-1⁺ | 20/80 | Δ | ○ | ⊚ |
| 2-2⁺ | 33/67 | ○ | ⊚ | ⊚ |
| 2-3⁺ | 67/33 | ○ | ⊚ | ⊚ |
| 2-4⁺ | 83/17 | ○ | ⊚ | ⊚ |
| 2-5⁺ | 96/4 | ○ | ⊚ | ⊚ |
| 2-6⁺ | 100/0 | Δ | ○ | Δ |
| Nothing | - | Δ | Δ | ⊚ |
| (Ethanol) | | | | |

| | | | | |
|---|---|---|---|---|
| (⁺) does not fall within the scope of the present invention | | | | |

As is clear from TABLE 18, it is understood that an excellent resistance to washing is exhibited and coloring retention is improved even in the case where the copolymer of the present invention is comprised into an acidic hair color.

Also, in the case where the percentage of Monomer (A) in the copolymer is too small or too large, resistance to washing becomes poor and coloring retention tends to be lowered. Accordingly, the percentage of Monomer (A) in the copolymer is preferably 25 to 99 wt% and, more preferably, 40 to 85 wt%.

### Test Example 2-3

Next, by using the copolymer having a monomer composition of TABLE 21, a temporary and acidic hair color were prepared according to the following prescriptions 2-1 and 2-2, respectively. These hair colors had been tested in the same way as Test Example 2-1. A compounding amount of each copolymer was 2 wt%. Also, in coloring retention test, smoothness and texture after applying the sample, washing with water and natural drying were evaluated according to the evaluation standard of the above-mentioned Test Example 1-3.

**TABLE 19**

| Temporary hair color (prescription 2-1, copolymer 2 wt%) | | | | | | |
|---|---|---|---|---|---|---|
| Copolymer | Monomer(wt%) | | | Coloring Retention | Smoothness | Flaking |
| | A2 | B1 | C* | | | |
| 2-7⁺ | 67 | 33 | - | ○ | ○ | ⊚ |
| 2-8⁺ | 50 | 10 | 40(C1) | ⊚ | ⊚ | ⊚ |
| 2-9 ⁺ | 50 | 10 | 40(C2) | ⊚ | ⊚ | ⊚ |
| 2-10⁺ | 50 | 40 | 10(C3) | ⊚ | ⊚ | ⊚ |
| 2-11⁺ | 60 | 35 | 5(C4) | ⊚ | ⊚ | ⊚ |
| 2-12⁺ | 50 | - | 50(C2) | ○ | ⊚ | ⊚ |
| 2-13⁺ | 25 | - | 75(C4) | Δ | ⊚ | ⊚ |

| | | | | | | |
|---|---|---|---|---|---|---|
| (+) does not fall within the scope of the present invention | | | | | | |

**TABLE 20**

| Acidic hair color (prescription 2-2, copolymer 2 wt%) | | | | | | |
|---|---|---|---|---|---|---|
| Copolymer | Monomer(wt%) | | | Coloring Retention | Smoothness | Flaking |
| | A2 | B1 | C* | | | |
| 2-7⁺ | 67 | 33 | - | ○ | ⊚ | ⊚ |
| 2-8⁺ | 50 | 10 | 40(C1) | ⊚ | ⊚ | ⊚ |
| 2-9⁺ | 50 | 10 | 40(C2) | ⊚ | ⊚ | ⊚ |
| 2-10⁺ | 50 | 40 | 10(C3) | ⊚ | ⊚ | ⊚ |
| 2-11⁺ | 60 | 35 | 5(C4) | ⊚ | ⊚ | ⊚ |
| 2-12⁺ | 50 | - | 50(C2) | ⊚ | ⊚ | ⊚ |
| 2-13⁺ | 25 | - | 75(C4) | ○ | ⊚ | ⊚ |

| | | | | | | |
|---|---|---|---|---|---|---|
| * The inside of ( ) represents Monomer (C) used. (+) does not fall within the scope of the present invention | | | | | | |

As is clear from TABLES 19 and 20, along with coloring retention of the hair color, feeling of use of hair after treatment could be improved by introducing Monomer (C) into the copolymer. Although copolymers 2-12 and 2-13 which were consisted of Monomers (A) and (C) were difficult to treat due to its stickiness, coloring retention and smoothness were fully satisfied.

Consequently, the percentage of Monomer (C) in the composition is preferably 1 wt% or more and, more preferably, 5 wt% or more. Also, Monomer (B) in the copolymer contributed to prevent stickiness of the copolymer. Accordingly, it is indicated that the percentage of Monomer (B) is preferably 1 wt% or more and, more preferably, 10 wt% or more.

**TABLE 21**

| Temporary hair color (prescription 2-1, copolymer 2 wt%) | | | | | | |
|---|---|---|---|---|---|---|
| Copolymer | Monomer (wt%) | | | | Coloring Retention | Texture |
| | A2 | B1 | C4 | D1 | | |
| 2-11⁺ | 60 | 35 | 5 | - | ⊚ | Δ |
| 2-14⁺ | 60 | 35 | 5 | 10 | ⊚ | ⊚ |
| 2-15⁺ | 60 | 35 | 5 | 20 | ○ | ⊚ |
| 2-16⁺ | 60 | 35 | 5 | 50 | ○ | ⊚ |
| 2-17⁺ | 60 | 35 | 5 | 100 | × | ⊚ |

| | | | | | | |
|---|---|---|---|---|---|---|
| (⁺) does not fall within the scope of the present invention | | | | | | |

As is clear from TABLE 21, good texture and moist touch can be given to hair in the case where the copolymer which is consisted of Monomers (A) to (D). AJso, when a percentage of Monomer (D) is higher, coloring retention effect tends to be lowered because hydrophilic nature of the cross-linked coat becomes high. This tendency was the same for the acidic hair color. Accordingly, the percentage of Monomer (D) is 1 to 100 wt% and, preferably, 5 to 50 wt% with respect to a whole amount of Monomers (A) to (C) in the composition.

As mentioned above, in the case where the cosmetics comprising the copolymer containing reactive silyl groups, along with a color material such as pigment or acidic dye, is applied on the hair and the cross-linking reaction is effected, the cosmetic is excellent in water-repellent and resistance to washing and has no adhesion against the object to be contacted, and retention of hair color is remarkably improved. Also feeling of use is not stiffened and no flaking occurs. Also, even in the case where the cosmetic of the present invention is applied as a make up cosmetic, the same effects can be exhibited and makeup retention is considerably improved.

Such effect can be considered as follows. A cross-linked coat strongly coats the applied portion in network state with enveloping the coloring material such as pigment or acidic dye. Accordingly, a coat excellent in water-repellency and resistance to washing can be formed.

Therefore, the composition comprising the copolymer containing reactive silyl groups and pigment and/or acidic dye is useful as a cosmetic and, in particular, as a hair color such as temporary or acidic hair color and a make up cosmetic.

Preferred examples of the copolymer to be comprised to such cosmetics can include the copolymers shown by Formula (X).

As a monomer composition of the copolymer, the percentage of Monomer (A) in the copolymer is preferably 25 to 99 wt% and, more preferably, 40 to 85 wt%. In the case where the percentage of Monomer (A) is too small, water-repellency and resistance to washing may be low and improvement effect of makeup retention may not be exhibited sufficiently, since the part for cross-linking reaction is few. On the other hand, water-repellency and resistance to washing and improvement effect of makeup retention tends to be lowered even in the case where the percentage of Monomer (A) is 100 wt%. Also, flaking is easy to occur. This can be considered that cross-linking reaction is not progressed neatly and a large amount of the unreacted part is remained on the copolymer because cross-linking reaction part is too much to be close.

Monomer (B) adjusts the ratio of Monomer (A). Also, it contributes to water-repellency and inhibits flaking. The percentage of Monomer (B) is 1 wt% or more and, preferably 10 wt% or more in the copolymer. The percentages of other monomers are decreased and the copolymer becomes to have poor solubility against alcohol type solvent in the case where Monomer (B) is too much. Therefore, the percentage of Monomer (B) in the copolymer is 75 wt% or less and, preferably, 60 wt% or less.

Monomer (C) has a siloxane part. Accordingly, Monomer (C) can improve water-repellency and resistance to washing of the cross-linked coat and make feeling of use smooth and favorable. Also, Monomer (C) can inhibits flaking. The percentage of Monomer (C) is 1 wt% or more and, preferably, 5 wt% or more in the copolymer of the present invention. However, when the percentage of Monomer (C) is too large, the percentages of the other monomers become relatively low and resistance to washing tends to be poor. Therefore, the percentage of Monomer (C) in the copolymer is 70 wt% or less and, preferably, 60 wt% or less.

Also, when the copolymer containing Monomer (D) as a constitutive monomer is treated, good texture and moist feeling of use can be given to hair. However, when the percentage of Monomer (D) is too large, water-repellence, resistance to washing and the like become to be poor because hydrophilic nature of the coat becomes high due to amine portion contained in Monomer (D). The percentage of Monomer (D) is 1 to 100 wt% and, preferably, 5 to 50 wt% with respect to a whole amount of Monomers (A) to (C).

Although a compounding amount of the copolymer is not restricted in particular, it is preferably 0.1 to 10 wt% and, more preferably, 1 to 5 wt%. In the case where the compounding amount is too less, the sufficient effect can not be obtained at one time treatment. On the contrary, applicability, spreadability and the like become poor and flaking may occur in the case where the compounding amount is too much.

The pigment and acidic dye, which are comprised into the cosmetic of the present invention is not restricted in particular as long as those are generally compounded into cosmetics.

As for the acidic dye, in specific, a legal color which is noticed on "Ministrial Ordinance to Determine Tar Color which is Usable for Medicaments and the like" and allowed to use for coloring medicaments, quasi-drugs and cosmetics and which is not shown harmful effect to the human body, can be used. For example, Red No. 3 (erythrosine), Red No. 102 (new coccin), Red No. 106 (acid red), Red No. 201 (lithol rubine B), Red No. 227 (fast acid magenta), Red No. 230 (1) (erythrosine YS), Red No. 203 (2) (erythrosine YSK), Red No. 231 (phloxine BK), Red No. 232 (rose bengal K), Red No. 401 (violamine R), Red No. 502 (ponceau 3R), Red No. 503 (ponceau R), Red No. 504 (ponceau SX), Red No. 506 (fast red S), Yellow No. 202 (2) (uranine K), Yellow No. 4 (tartrazine), Yellow No. 402 (polar yellow 5G), Yellow No. 403 (1) (naphthol yellow S), Yellow No. 406 (metanil yellow), Green No. 3 (fast green FCF), Green No. 201 (alizanine cyanine green F), Green No. 204 (pyranine conc), Green No. 205 (light green SF yellowish), Green No. 401 (naphthol green B), Green No. 402 (guinea green B), Blue No. 1 (brilliant blue FCF), Blue No. 2 (indigo carmine), Blue No. 202 (patent blue NA), Blue No. 205 (alfazurine FG), Brown No. 201 (resorcine brown), Purple No. 401 (alizurol purple), Black No. 401 (naphthol blue black), and the like can be used.

Pigments used in the present invention include an organic, inorganic and lake pigments.

Example of an organic pigment include, in addition to azo pigment, indigo pigment and phthalocyanine pigment, an organic dye such as β -carotene, carthamin or cochineal.

Example of an inorganic pigment include, in addition to a coloring pigment such as red iron oxide, yellow iron oxide, black iron oxide, chromium oxide, ultramarine, iron blue and carbon black, an extender pigment such as mica, talc or kaolin, a white pigment such as titanium dioxide or zinc oxide, a pearl pigment such as titanium mica or fish scale flake and a specific functional pigment such as boron nitride, a photochromic pigment, synthetic mica or hybrid fine powder.

Example of a lake pigment include, in addition to a lake pigment such as Red Nos. 202, 204, 206, 207 or 220, a dye lake pigment such as aluminium lake.

Although a compounding amount of the copolymer in the cosmetic in accordance with the present invention is not restricted in particular, it is preferably 0.1 to 10 wt% and, more preferably, 1 to 5 wt%. In the case where the compounding amount is too less, the sufficient effect can not be obtained at one time treatment. On the contrary, applicability, spreadability and the like become poor and flaking may occur in the case where the compounding amount is too much.

A compounding amount of the pigment and acidic dye can be properly determined according to the required colorability and is not restricted in particular. However, the compounding amount is usually 0.01 to 20 wt% and, preferably, 0.1 to 10 wt%. Secondary adhesion is easy to occur in the case where the pigment or dye is too much.

A cosmetic preparation in accordance with the present invention can be, for example, in addition to a hair color such as color spray, color crayon or color mousse, a makeup cosmetic such as nail enamel, mascara or eye brow.

The present inventors further studied about the effects in the case where the other film-forming ingredients were used in the cosmetic which was comprised the copolymer and pigment and/or acidic dye. As for a film-forming ingredient, siliconized pullulan (substitution degree 1.7, molecular weight of pullulan as a starting material about 200,000) was used.

### Test Example 2-4

### Prescription 2-3 (Temporary hair color):

| | |
|---|---|
| Copolymer | 5 wt% |
| Siliconized pullulan | 1 |
| Carbon black | 1 |
| Ethanol | 22.8 |
| Light isoparaffin | 70 |
| Silicone rubber | 0.2 |
| Perfume | q. s. |
| Antiseptic | q. s. |

A temporary hair color was prepared according to the above-mentioned prescription 2-3. Also, a hair color, which comprised ethanol instead of siliconized pullulan, was prepared as a control. The test had been conducted in the following method by using each hair color as a sample.

### (Coloring retention)

Each sample was applied to white hair and the hair was left for 3 minutes. Then the hair was washed with water and dried naturally. Thereafter, the hair was washed with a commercially available shampoo and was dried naturally every day, and coloring retention after 10 days was evaluated by visual inspection. Evaluation standard is as follows.
⊚ : Very good as compared with the control.
○ : Equal to or slightly good as compared with the control.
Δ : Slightly inferior as compared with the control.
× : Inferior as compared with the control.

### (Smoothness)

Smoothness at 1st and 10th days of the above-mentioned coloring retention test was evaluated by touch. Evaluation standard is as follows.
⊙ : Very good as compared with the control.
○ : Equal to or slightly good as compared with the control.
Δ : Slightly inferior as compared with the control.
× : Inferior as compared with the control.

### (Flaking on repeated application)

Flaking was evaluated in the same way as the method of the above-mentioned Test Example 1-4.

**TABLE 22**

| Copolymer | Monomer (wt%) | | | | Coloring | Smoothness | | Flaking on |
|---|---|---|---|---|---|---|---|---|
| | A2 | B1 | C* | D1 | Retention | 1st | 10th | Repeated Application |
| 2-19 | 60 | 35 | 5(C3) | 20 | ⊚ | ⊚ | ⊚ | ⊚ |
| 2-20⁺ | 50 | 40 | 10(C1) | - | ○ | ⊚ | ⊚ | ⊚ |
| 2-21⁺ | 50 | 25 | 25(C2) | - | ○ | ⊚ | ⊚ | ⊚ |
| 2-22⁺ | 67 | 37 | - | - | ○ | ⊚ | ⊚ | ⊚ |
| 2-23⁺ | 50 | - | 50(C3) | - | ○ | ⊚ | ⊚ | ⊚ |

| | | | | | | | | |
|---|---|---|---|---|---|---|---|---|
| (⁺) does not fall within the scope of the present invention | | | | | | | | |

As is clear from TABLE 22, in the case where siliconized pullulan was used together with the copolymer, the effect of coloring retention can be made equal to or better than the case where only the copolymer was used. Touch also can be made more smooth as compared with the case of using the copolymer only, and in particular, the effect with respect to the damaged hair is remarkable. These effects were maintained even when shampoo was repeated. Further, though flaking on repeated application may occur in the case where only the copolymer was used, it can be inhibited by using siliconized pullulan together with. Such effects were the same for the acidic hair color.

**TABLE 23**

| Ingredient | Amount | | | | | | | |
|---|---|---|---|---|---|---|---|---|
| Copolymer 2-19 | 5 | 5 | 5 | 2 | 2 | 1 | 0.1 | - |
| Siliconized pullulan | - | 0.1 | 0.5 | 0.5 | 2 | 5 | 5 | 5 |
| Coloring Retention | - | ○ | ⊚ | ⊚ | ⊚ | ○ | Δ | Δ |

| Smoothness | | | | | | | | |
|---|---|---|---|---|---|---|---|---|
| 1 st day | - | ○ | ⊚ | ⊚ | ⊚ | ⊚ | ⊚ | ⊚ |
| 10th day | - | ○ | ⊚ | ⊚ | ⊚ | ⊚ | ⊚ | ⊚ |
| Flaking on | Δ | ⊚ | ⊚ | ⊚ | ⊚ | ⊚ | ⊚ | ⊚ |
| Repeated Application | | | | | | | | |

TABLE 23 shows the result that the test was conducted in the same way as Test Example 2-4 with changing the ratio of the copolymer and siliconized pullulan. The changing amount of the copolymer and pullulan was adjusted with ethanol.

Coloring retention tends to be lowered in the case where the copolymer is not much. On the other hand, coloring retention and smoothness are not sufficiently improved and flaking on repeated application may occur in the case where siliconized pullulan is not much. Accordingly, the ratio between the copolymer and siliconized pullulan is preferably 100 : 1 and, more preferably, 50 : 1- 1 : 1 by weight.

As described in the above, by using the copolymer containing reactive silyl groups and the siliconized polysaccharide together with the pigment and/or acidic dye, the cosmetic that coloring retention and feel of use are extremely favorable and flaking on repeated application is not much, can be obtained.

In the case where only the siliconized polysaccharide was used, coloring retention effect were low as compared with the case where only the copolymer was used. Therefore, it can be considered that these effects are improved by using the siliconized polysaccharide together with the copolymer because at the time of forming the cross-linked coat the siliconized polysaccharide is included into the coat and the cross-linked coat and the siliconized polysaccharide are synergistically contributed.

### Examples 2-1 to 2-3 Color Spray

The test had been conducted in the same manner as the above-mentioned Test Example 2-1 by using the color sprays of TABLE 30. Every example had high resistance to washing to be excellent in coloring retention as compared with Comparative Example 2-1

**TABLE 30**

| Ingredient | Example 2-1 | Example 2-2 | Example 2-3 | Comp. Ex. 2-1 |
|---|---|---|---|---|
| (1) Copolymer 2-11⁺ | 1 | - | - | - |
| (2) Copolymer 2-13⁺ | - | 1 | - | - |
| (3) Copolymer 2-14 | - | - | 1 | - |
| (4) Polyvinylpyrrolidone/ | - | - | - | 1 |
| vinyl acetate copolymer | | | | |
| (5) Resorcine brown | 1 | 1 | 1 | 1 |
| (6) Ethanol | 48.2 | 48.2 | 48.2 | 48.2 |
| (7) Benzyl alcohol | 5 | 5 | 5 | 5 |
| (8) Citric acid | 0.3 | 0.3 3 | 0.3 | 0.3 |
| (9) POE (100) hydrogenated castor oil | 1 | 1 | 1 | I |
| (10) Highly polymerized | 0.5 | 0.5 | 0.5 | 0.5 |
| dimethylpolysiloxane | | | | |
| (11) Dimethyl polysiloxane (5cps) | 3 | 3 | 3 | 3 |
| (12) LPG (3.5kg/cm² at 20°C) | 40 | 40 | 40 | 40 |
| (13)Perfume | q.s. | q.s. | q.s. | q.s. |
| Coloring Retention | ⊚ | ⊚ | ⊚ | × |
| Smoothness | ⊚ | ⊚ | ⊚ | × |

| | | | | |
|---|---|---|---|---|
| (⁺) does not fall within the scope the present invention | | | | |

### (Manufacturing Process)

The ingredients (1) to (11) and (13) were stirred and mixed in uniform. Then the mixture and the ingredient (12) were filled into an aerosol container, successively to obtain a color spray.

### Example 2-4 Nail Enamel

Nail enamels were prepared according to the following prescription 2-6 by using the above-mentioned Copolymers 2-11, 12 and 14. The nail enamel was applied on the nails of female panel. After drying, the nail was washed with water. Thereafter, the nail was observed in every day. Crack or peeling was observed within 3 days in the case where ethanol was comprised instead of the copolymer. However, crack or peeling of control was absolutely not observed for 10 days or more and coloring retention was maintained in the case where the copolymer was comprised.

### Prescription 2-6:

| | |
|---|---|
| Nitrocellulose | 10 wt% |
| Alkyd resin | 10 |
| Acetyltriethyl citrate | 5 |
| Ethyl acetate | 10 |
| Butyl acetate | 5 |
| Copolymer | 5 |
| Ethanol | 55 |
| Rhodamine B | q.s. |
| Suspending agent | q.s. |

### (Manufacturing process)

Rhodamine B was added to a part of alkyd resin and a part of acetyl triethyl citrate and the mixture was fully kneaded. The other ingredients were mixed and dissolved, and the former mixture was added thereto. The mixture was dispersed in uniform to obtain a nail enamel.

### Example 2-5 Color Spray

| | |
|---|---|
| Red iron oxide | 1 wt% |
| Copolymer 2-19 | 4 |
| Siliconized pullulan | 1 |
| Silicone rubber | 0.2 |
| Light isoparaffin | 70 |
| Ethanol | 23.8 |
| Perfume | q.s. |
| Antiseptic | q.s. |

### (Manufacturing Process)

After silicone rubber and siliconized pullulan were dissolved into light isoparaffin, a stock solution of a spray was prepared by adding the ethanol solution of Copolymer 2-19 and the other ingredients to the former solution. 70 parts of the stocked solution was filled into a can and a valve was mounted. 30 parts of dimethyl ether gas was filled into the can to obtain a color spray.

The color spray had favorable coloring retention and feeling of use and inhibited flaking on repeated application as compared with the color spray that siliconized pullulan was not comprised.

### < Application for Other Materials To Be Treated >

The copolymer of the present invention has an excellent coat-forming property and resistance to washing. Accordingly, the present inventors studied about the effect with respect to the material except for the hair or skin, e.g., fiber, paper, glass and the like.

### Test Example 3-1

First, the test was conducted according to the following method so as to investigate the effect to fiber.

### (Water-repellency)

5% ethanol solution of each copolymer was sprayed on a cotton shirt and the shirt was dried naturally. A few drops of water were dropped onto the shirt and its water-repellency was evaluated with visual sensation. Also, a set of washing the shirt with a washing machine by using a commercially available household detergent and drying naturally was repeated 10 times. Thereafter, water-repellency was evaluated in the same way as mentioned above.

### Evaluation standard

⊚ : Extremely favorable
O : Favorable
Δ : Slightly unfavorable
× : Unfavorable

### (Smoothness)

2g of 5% ethanol solution of each copolymer was applied on 10g of a sheet of cotton fabric and dried naturally. Smoothness of this fabric had been evaluated by 20 of professional panel with touch.

### Evaluation standard

⊚ : 15 or more answered favorable.
○ : 10 to 14 answered favorable.
Δ : 5 to 9 answered favorable.
× : 4 or less answered favorable.

### (Suitability for sizing)

5% ethanol solution of the tested copolymer was sprayed on a cotton shirt and was hardened by ironing with a steam iron. The state of shirt immediately after ironing has been evaluated with touch. Further, suitability for 10 times of sizing after washing and drying in the similar manner to the water-repellency test mentioned above.

### Evaluation standard

⊚ : Extremely favorable.
O : Favorable.
Δ : Slightly unfavorable.
× : Unfavorable.

### (Crease-smoothing property/crease resistance)

In the similar manner to the above-mentioned suitability for sizing test, the states of crease after ironing and 10 times of washing and drying were judged with visual sensation and thereby crease-smoothing property/crease resistance was evaluated.

### Evaluation standard

⊚ : Extremely favorable.
○ : Favorable.
Δ : Slightly unfavorable.
× : Unfavorable.

### (Unsusceptibility to fouling)

A drop of coffee was dropped onto the shirt that was treated in the same way as the above-mentioned water-repellency test. Attachment degree of fouling after wiping the drop with another fabric was judged with visual sensation. Thereby, unsusceptibility to fouling was evaluated.

### Evaluation standard

⊚ : Extremely favorable
O : Favorable
Δ : Slightly unfavorable
×: Unfavorable

### (Texture)

2g of 5% ethanol solution of each copolymer was applied on 109 of a sheet of cotton fabric and dried naturally. Texture of this fabric had been evaluated by 20 of professional panel with touch.

### Evaluation standard

⊚ : 15 or more answered favorable.
O : 10 to 14 answered favorable.
Δ 5 to 9 answered favorable.
× : 4 or less answered favorable.

Next, the copolymer having Monomers (C) and (D) along with Monomers (A) and (B) was tested.

**TABLE 37**

| Copolymer | Monomer (wt%) | | | Water-repellency | | Unsusceptibility | Smoothness |
|---|---|---|---|---|---|---|---|
| | A2 | B1 | C2 | just after treatment | after washing | to Fouling | |
| 3**-**1⁺ | 10 | 40 | 50 | ○ | × | ○ | ⊚ |
| 3-2⁺ | 20 | 40 | 40 | ○ | × | ⊚ | ⊚ |
| 3-3⁺ | 45 | 50 | 5 | ⊚ | ⊚ | ⊚ | ⊚ |
| 3-4⁺ | 50 | 25 | 25 | ⊚ | ⊚ | ⊚ | ⊚ |
| 3-5⁺ | 50 | 10 | 40 | ⊚ | ⊚ | ⊚ | ⊚ |
| 3-6⁺ | 55.6 | 33.3 | 11.1 | ⊚ | ⊚ | ⊚ | ⊚ |
| 3-7⁺ | 60 | 35 | 5 | ⊚ | ⊚ | ⊚ | ⊚ |
| 3-8⁺ | 71.4 | 14.3 | 5 | ⊚ | ⊚ | ⊚ | ⊚ |
| 3-9⁺ | 85 | 10 | 5 | ○ | Δ | ○ | ⊚ |

| | | | | | | | |
|---|---|---|---|---|---|---|---|
| (+) does not fall within the scope of the present invention | | | | | | | |

As is clear from TABLE 37, in the case where the copolymer consisting of Monomers (A) to (C) are treated, water-repellency, resistance to washing and unsusceptibility to fouling can be considerably improved as compared with the copolymer consisting of Monomers (A) and (B). Also, the result of the treated shirts was very smooth to be extremely favorable. In order to obtain such effects, the percentage of Monomer (C) in the copolymer is I wt% or more and, preferably, 5 wt% or more.

Copolymer consisting of Monomers (A) and (C) has stickiness. As for Monomer B, it was indicated that a presence of Monomer (B) inhibited stickiness caused by Monomer (C) to give a moderate nature to the coat. The percentage of Monomer (B) in the copolymer is 5 wt% or more and, preferably, 10 wt% or more.

**TABLE 38**

| Copolymer | Monomer (wt%) | | | Water-repellency | | Unsusceptibility | Smoothness |
|---|---|---|---|---|---|---|---|
| | A2 | B1 | C* | just after | after | to Fouling | |
| | | | | treatment | washing | | |
| 3-10⁺ | 50 | 10 | 40(C 1) | ⊚ | ⊚ | ⊚ | ⊚ |
| 3-11⁺ | 60 | 35 | 5(C3) | ⊚ | ⊚ | ⊚ | ⊚ |
| 3-12⁺ | 60 | 35 | 5(C4) | ⊚ | ⊚ | ⊚ | ⊚ |
| 3-13⁺ | 50 | 25 | 25(C4) | ⊚ | ○ | ⊚ | ⊚ |

| | | | | | | | |
|---|---|---|---|---|---|---|---|
| * The inside of ( ) represents Monomer (C) used. (⁺) does not fall within the scope of the present invention | | | | | | | |

As is clear from TABLE 38, even when Monomer (C) in the copolymer was Monomer C1, C3 or C4, the same effect could be obtained in the case where Monomer (C) is Monomer C2.

**TABLE 39**

| Copolymer | Monomer (parts by weight) | | | | | Water-repellency | | Texture |
|---|---|---|---|---|---|---|---|---|
| | A 1 | B1 | C3 | C4 | D1 | just after treatment | after washing | |
| 3-11⁺ | 60 | 35 | 5 | - | - | ⊚ | ⊚ | Δ |
| 3-12⁺ | 60 | 35 | - | 5 | - | ⊚ | ⊚ | Δ |
| 3-14 | 60 | 35 | 5 | - | 10 | ⊚ | ⊚ | ⊚ |
| 3-15 | 60 | 35 | - | 5 | 10 | ⊚ | ○ | ⊚ |
| 3-16 | 60 | 35 | - | 5 | 20 | ⊚ | ○ | ⊚ |
| 3-17 | 60 | 35 | - | 5 | 50 | ○ | ○ | ⊚ |
| 3-18 | 60 | 35 | - | 5 | 100 | Δ | × | ⊚ |

| | | | | | | | | |
|---|---|---|---|---|---|---|---|---|
| (⁺) does not fall within the scope of the present invention | | | | | | | | |

As is clear from TABLE 39, the cross-linked coat of the copolymer consisting of Monomers (A) to (D) can gives fiber good texture and moisture touch. In the case
where Monomer (D) is too much, water-repellency and resistance to washing tend to be deteriorated since hydrophilic property of the cross-linked coat becomes high. Accordingly, the percentage of Monomer (D) is I to 100 wt% and, preferably, 5 to 50 wt% with respect to a whole amount of Monomers (A) to (C) in the copolymer.

**TABLE 40**

| | Copolymer | | | | | Untreated |
|---|---|---|---|---|---|---|
| | 3-4⁺ | 3-10⁺ | 3-11⁺ | 3-13⁺ | 3-15 | |
| Suitability for Sizing | | | | | | |
| (just after treatment) | ⊚ | ⊚ | ⊚ | ⊚ | ⊚ | × |
| (after washing) | ⊚ | ⊚ | ⊚ | ○ | ○ | × |

| Crease-smoothing property/crease resistance | | | | | | |
|---|---|---|---|---|---|---|
| (just after ironing) | ⊚ | ⊚ | ⊚ | ⊚ | ⊚ | × |
| (after washing) . | ⊚ | ○ | ⊚ | ○ | ○ | × |

| | | | | | | |
|---|---|---|---|---|---|---|
| (⁺) does not fall within the scope of the present invention | | | | | | |

As is clear from TABLE 40, the cross-linked coat of the copolymer in accordance with the present invention exhibits an excellent effect in suitability for sizing and crease-smoothing property/crease resistance with respect to fiber.

**TABLE 41**

| | Copolymer | | | | | Untreated |
|---|---|---|---|---|---|---|
| | 3-4⁺ | 3-10⁺ | 3-11⁺ | 3-13⁺ | 3-15 | |
| Water-repellency (just after treatment) | ⊚ | ⊚ | ⊚ | ⊚ | ⊚ | × |
| Unsusceptibility to Fouling | ⊚ | ⊚ | ⊚ | ⊚ | ⊚ | × |

| | | | | | | |
|---|---|---|---|---|---|---|
| (⁺) does not fall within the scope of the present invention | | | | | | |

TABLE 41 shows the result of water-repellency test and unsusceptibility to fouling test conducted in the same way by using paper instead of a cotton shirt. As a result, it is understood that high water-repellency and unsusceptibility to fouling can be obtained even in the case where the copolymer of the present invention is treated on paper.

**TABLE 42**

| | Copolymer | | | | | Untreated |
|---|---|---|---|---|---|---|
| | 3-4⁺ 3-10⁺ 3-11⁺ 3-13⁺ 3-15 | | | | | |
| Angle of contact | 96.0 | 96.4 | 92.0 | 97.0 | 94.6 | 9.7 |
| Angle of inclination | 13.0 | 11.0 | 14.2 | 15.0 | 13.8 | -* |

| | | | | | | |
|---|---|---|---|---|---|---|
| * Impossible to measure because waterdrop was remained. (⁺) does not fall within the scope of the present invention | | | | | | |

TABLE 42 is the result when hydrophobic degree of glass is investigated by using a glass plate in the place of a cotton shirt and by treating in the similar manner to the above-mentioned water-repellency test. Hydrophobic degree was evaluated by the angles of contact and inclination with respect to water. A drop of water was dropped onto a glass and the angle of contact was measured. Also, a glass was gradually inclined and the angle at just when the waterdrop started to move was measured as the angle of inclination. The larger the angle of contact is and the smaller the angle of inclination is, the larger hydrophobic degree can be evaluated.

As a result, the glass plate treated with the copolymer in accordance with the present invention has very large angle of contact to water as compared with the untreated glass plate. On the other hand, the angle of inclination is too small. Accordingly, it was indicated that the copolymer of the present invention can gives the glass plate very high hydrophobic ability.

As mentioned above, the copolymer of the present invention formed the cross-linked coat which was excellent in water-repellency and unsusceptibility to fouling by cross-linking reaction on the treated material. Also, the coat is very hard to be removed even with water or detergent to these effects exhibit continuously. Accordingly, the copolymer can be used as a water-repellent or a stain proofing agent for various materials. Also, the copolymer of the present invention is preferable as a treatment for fiber such as water-repellent for fiber, size for fiber or crease-resistant agent because the cross-linked coat causes very few flaking and when the copolymer is applied to a treatment for fiber, the cross-linked coat has suitability for sizing, crease-resistance, and very excellent feeling of use.

A preferable example of the copolymer used for such water-repellent or treatment for fiber can be shown by Formula (X).

As a monomer composition of the copolymer, the percentage of Monomer (A) in the copolymer is preferably 25 to 85 wt% and, more preferably, 40 to 75 wt%. In the case where the percentage of Monomer (A) is too small, the effect such as water-repellency or resistance to washing may not be sufficiently exhibited since the part for the cross-linking reaction is few. On the contrary, water-repellency or resistance to washing tends to be deteriorated in the case where the percentage of Monomer (A) is too large. It can be considered that this is the result that cross-linking reaction is not progressed neatly and a large amount of the unreacted part is remained on the cross-linked copolymer because the part for cross-linking reaction is too much to be close. Also, feeling of use may be worse and flaking may be caused.

Monomer (B) contributes to water-repellency of the cross-linked coat. Also, Monomer (B) inhibits flaking and stickiness caused by Monomer (C) to adjust the nature of the cross-linked coat. The percentage of Monomer (B) is 5 wt% or more and, preferably, 10 wt% or more in the copolymer. The percentages of other monomers are decreased and the copolymer becomes to have poor solubility against alcohol type solvent in the case where the percentage of Monomer (B) is too large. Therefore, the percentage of Monomer (B) is 75 wt% or less and, preferably, 60 wt% or less.

Monomer (C) has a siloxane part. Accordingly, Monomer (C) can- remarkably improve water-repellency of the cross-linked coat and can giving fiber smoothness to make the result favorable. Monomer (C) also inhibits flaking. The percentage of Monomer (C) is 1 wt% or more and, preferably, 5 wt% or more in the copolymer of the present invention. However, when the percentage of Monomer (C) is too large, the percentages of the other monomers become relatively small and resistance to washing tends to be poor. Therefore, the percentage of Monomer (C) is 70 wt% or less and, preferably, 60 wt% or less.

Also, when the copolymer, which comprises Monomer (D) together with Monomers (A) to (C) as constitutive monomers, is treated, smoothness and good texture can be given to fiber and moist feeling of use can be obtained. However, in the case where Monomer (D) is too much, water-repellency, resistance to washing and the like are inferior because hydrophilic nature of the coating become high by amine portion contained in Monomer (D). The percentage of Monomer (D) is 1 to 100 wt% and, preferably, 5 to 50 wt% with respect to a whole amount of Monomers (A) to (C).

Although a concentration of the copolymer is not restricted in particular, it is preferably 0.1 to 10 wt% and, more preferably, 1 to 5 wt%. In the case where the amount of the copolymer is too little, the sufficient effect can not be obtained at once treatment. On the contrary, applicability, spreadability, feeling of use and the like become inferior and flaking may be caused in the case where the amount is too much.

The other ingredients can be comprised into the water-repellent or treatment for fiber of the present invention within the range that the effect of the present invention is not spoiled. Also, it is possible to form a colored coat having water-repellency when coloring materials such as pigment or dye are comprised therein. On the other hand, it is possible to prevent discoloring of the material to be treated when an ultraviolet absorber is comprised therein.

As for the material to be treated with the water-repellent and treatment for fiber of the present invention, fibrous materials having optional forms such as single fiber, spun yarn, random fiber, fabric and a final product are preferable. Such the final product can be, for example, shoes, an umbrella, a hat or cap, a ski suit, a shirt or a carpet. In the present invention, the fibrous material also includes leather, the other fibrous materials and products thereof such as fiber of paper or paper products. Also, in addition to the fibrous material, it is possible to treat the other materials such as glass plates, glass products, metal plates or metal products, that water-repellency and unsusceptibility to fouling are required thereto.

As a method for applying the water-repellent and treatment for fiber of the present invention, known methods, e.g., dipping method, roll coating method or spray method, may be used. Of course, instruments such as brush or comb can be used properly as occasion demands.

One of the most simple treatment method in the present invention can be, for example, the method that the material to be treated is dipped into or sprayed with a solution of the copolymer of the present invention and then the material is, optionally with squeezing, dried naturally or with heating. Also, in clothes such as a shirt, a method that the solution of the copolymer is sprayed on the cloth before ironing and then the cloth is pressed by iron or steam iron, is preferable.

Lastly, a synthetic example of the copolymer in accordance with the present invention is described.

### Synthetic Examples 1 to 6

In order that each of Monomers (A) to (D) has a ratio described in TABLE 43, the mixture of the monomers was dissolved into 100 ml of ethanol with the preparation amount. The solution was heated with stirring under nitrogen gas stream for hour at 70°C. 0.05g of potassium persulfate was added to the solution and the mixture was reacted overnight. The reaction mixture was cooled down to room temperature and concentrated under a vacuum. The residue was dissolved into 10ml of ethanol and the solution was added to 500ml of n-hexane. The deposits were collected to give the aimed copolymer.

**TABLE 43**

| Synthetic | Monomer (parts by weight) | | | | | | | Preparation | Yield |
|---|---|---|---|---|---|---|---|---|---|
| Examples | A2 | B1 | C1 | C2 | C3 | C4 | D1 | Amount (g) | (%) |
| 1 | 12 | 7 | - | - | - | 1 | 2 | 20 | 99.5 |
| 2 | 12 | 7 | - | - | - | 1 | 4 | 22 | 90.9 |
| 3 | 12 | 2 | - | - | - | 6 | 2 | 22 | 91.4 |
| 4 | 12 | 2 | - | - | - | 6 | 4 | 24 | 96.7 |
| 5 | 12 | 4 | - | - | - | 4 | 2 | 22 | 90.0 |
| 6 | 12 | 4 | - | - | - | 4 | 4 | 24 | 91.3 |

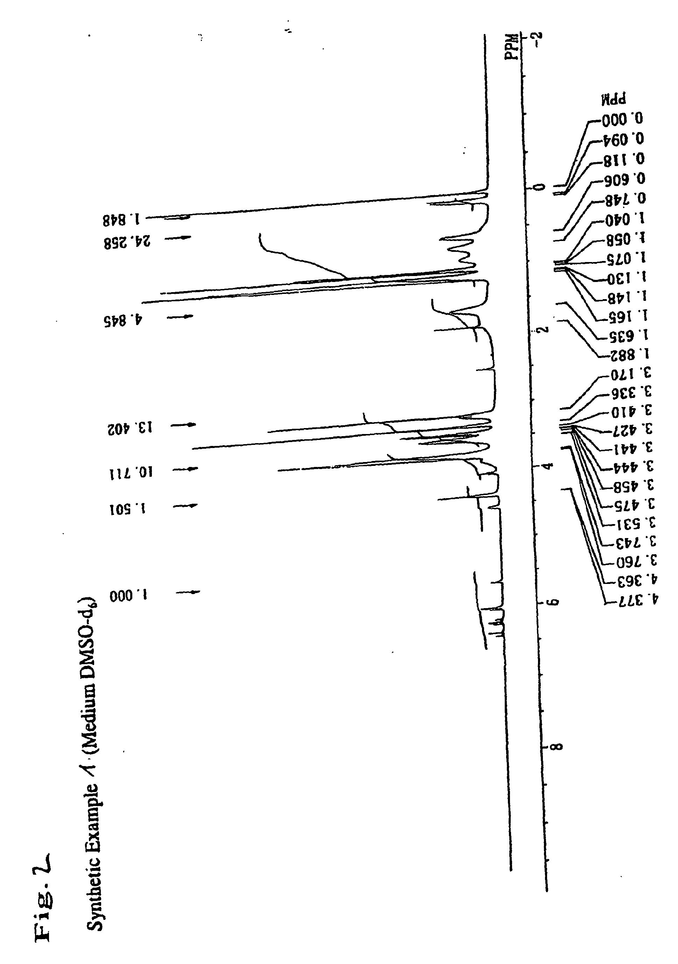

Fig. 2 shows NMR spectral data of the copolymer that was obtained by Synthetic Example 1. In these NMR spectral data (solvent CDCl₃ or DMSO-d₆) of the resulting copolymer, the peak of hydrogen atom of CH₂=C derived from the monomer used as a starting material, that the peak should be observed in 6-7 ppm or near, was not observed. As a result, generation of the copolymer was confirmed. Also, in the present invention, generation of the other copolymers was confirmed in the similar manner.

## Claims

1. A composition comprising a copolymer containing silyl groups wherein each silyl group has at least one reactive functional group bonded thereto,
wherein said copolymer comprises monomer (A) shown by the following Formula (1): 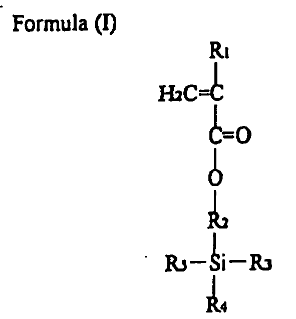 wherein R₁ is a hydrogen atom or methyl group; R₂ is an alkylene group having 1-6 carbon atoms; and R₃, R₄ and R₅ each is a reactive functional group which can cross-link molecules of the copolymer by hydrolyzing,
monomer (B) shown by the following Formula (II): 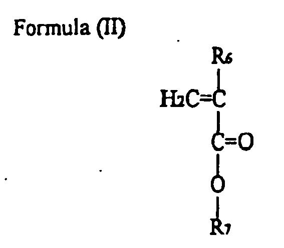 wherein R₆ is a hydrogen atom or methyl group; and R₇ is an alkyl group having 1-18 carbon atoms,
monomer (C) shown by the following Formula (III): 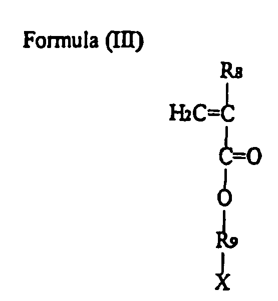 wherein R₈ is a hydrogen atom or methyl group; R₉ is an alkylene group having 1-6 carbon atoms; and X is a group expressed by any of the following Formulae (IV) to (VI): 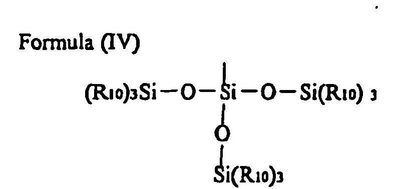 wherein R₁₀ is an alkyl group having 1-6 carbon atoms; 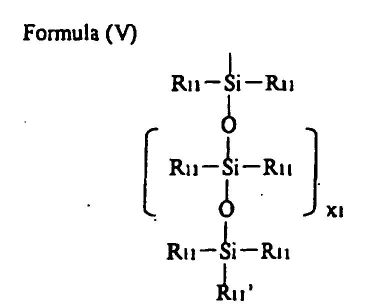 wherein R₁₁ and R₁₁' each is an alkyl group having 1-6 carbon atoms; and x₁ is an integer of positive number; and 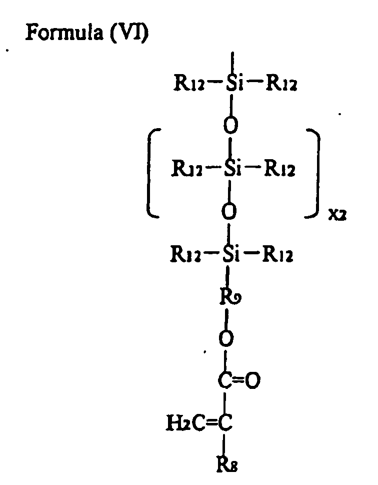 wherein R₈ and R₉ are the same as defined in Formula (III); R₁₂ is an alkyl group having 1-6 carbon atoms; and x₂ is an integer of positive number., and
monomer (D) shown by the following formula (VII): 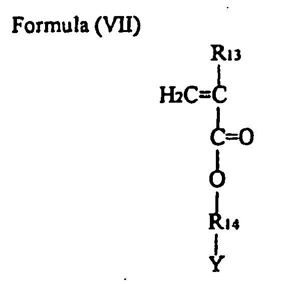 wherein R₁₃ is a hydrogen atom or methyl group; R₁₄ is an alkylene group having 1-6 carbon atoms; and Y is a group expressed by -N⁺(R₁₅)₃ or -N(R₁₅)₂, wherein R₁₅ is an alkyl group having 1-6 carbon atoms.

2. A composition according to claim 1, wherein the percentage of monomer (A) is 25 to 99 wt% in the copolymer.

3. A composition according to claim 1 or 2, wherein the percentage of monomer (B) is 1 wt% or more in the copolymer.

4. A composition according to any one of claims 1 to 3,
wherein the percentage of monomer (C) is 1 wt% or more in the copolymer.

5. A composition according to claim 1, wherein the percentage of monomer (D) is within the range of 1 to 100 parts by weight with respect to 100 parts by weight of a combination of monomer (A), monomer (B) and monomer (C) in the copolymer.

6. A composition according to any one of claims 1 to 5,
wherein each of R₃, R₄, and R₅ is an alkoxy group having 1-6 carbon atoms.

7. A composition according to any one of claims 1 to 6 which further comprises a pigment and/or an acidic dye.

8. A composition according to any one of claims 1 to 7, which further comprises a siliconized polysaccheride.

9. A copolymer comprising monomer (A) shown by formula (I) as defined in claim 1, monomer (B) shown by formula (II) as defined in claim 1, monomer (C) shown by formula (III) as defined in claim 1, monomer (D) shown by formula (VII) as defined in claim 1, as constitutive monomers.

10. The copolymer according to claim 9, wherein each of R₃, R₄ and R₅ is an alkoxy group having 1-6 carbon atoms.

11. Use of the composition according to any one of claims 1 to 8 for coating a surface.

12. Use of the composition according to any one of claims 1 to 8 for modifying the nature of hair.

13. Use of a composition according to any one of claims 1 to 8 as a cosmetic pack.

14. Use of a composition according to any of claims 1 to 8 as a water-repellent.

15. Use according to claim 14, wherein the percentage of monomer (A) is 25 to 85 wt% in the copolymer.

16. Use according to claim 14 or 15, wherein the percentages of monomer (B) and monomer (C) are 5 wt% or more and 1 wt% or more in the copolymer, respectively.

17. Use of a composition according to any one of claims 1 to 8 as a fibre-treating composition.

18. Use according to claim 17, wherein the percentage of monomer (A) is 25 to 85 wt% in the copolymer.

19. Use according to claim 17 or 18, wherein the percentages of monomer (B) and monomer (C) are 5 wt% or more and 1 wt% or more in the copolymer, respectively.

20. A coat-forming method comprising a step of: hydrolyzing the composition according to any of claims 1 to 8 on a material to be treated to cross-link molecules of the copolymer.

21. A coat-forming method according to claim 20, wherein the material to be treated is human hair or epidermis.

22. A coat-forming method according to claim 20, wherein the material to be treated is fiber, paper or glass.

23. A method for a pack comprising steps of: hydrolyzing the composition according to any of claims 1 to 6 on skin to cross-link molecules of the copolymer, and peeling the composition from the skin after drying.

## Patentansprüche

1. Zusammensetzung, die ein Silylgruppen enthaltendes Copolymer umfasst, wobei jede Silylgruppe mindestens eine daran gebundene reaktive funktionelle Gruppe aufweist,
wobei das Copolymer ein Monomer (A) der im folgenden angegebenen Formel (I): 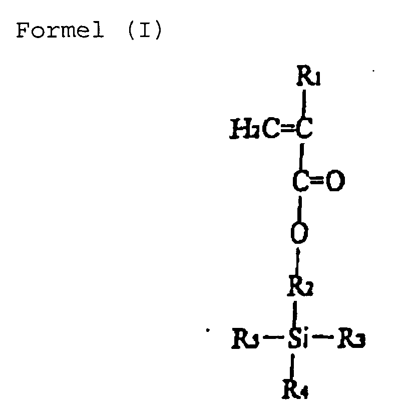 worin R₁ für ein Wasserstoffatom oder eine Methylgruppe steht; R₂ für eine Alkylengruppe mit 1-6 Kohlenstoffatomen steht; und R₃, R₄ und R₅ jeweils für eine reaktive funktionelle Gruppe, die Moleküle des Copolymers durch Hydrolyse vernetzen kann, stehen,
ein Monomer (B) der im folgenden angegebenen Formel (II): 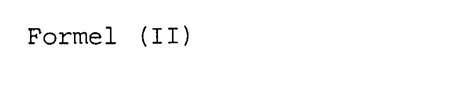 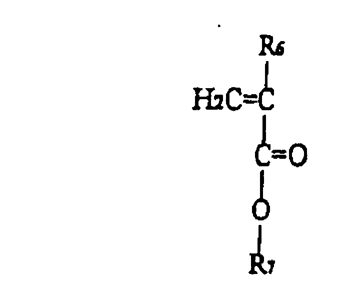 worin R₆ für ein Wasserstoffatom oder eine Methylgruppe steht; und R₇ für eine Alkylgruppe mit 1-18 Kohlenstoffatomen steht,
ein Monomer (C) der im folgenden angegebenen Formel (III) : 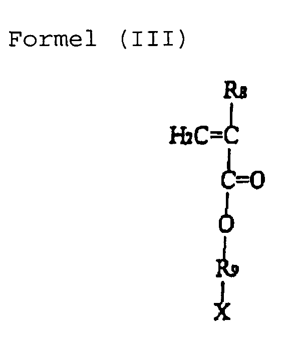 worin R₈ für ein Wasserstoffatom oder eine Methylgruppe steht; R₉ für eine Alkylengruppe mit 1-6 Kohlenstoffatomen steht; und X für eine Gruppe steht, die durch eine der im folgenden angegebenen Formeln (IV) bis (VI) ausgedrückt wird: 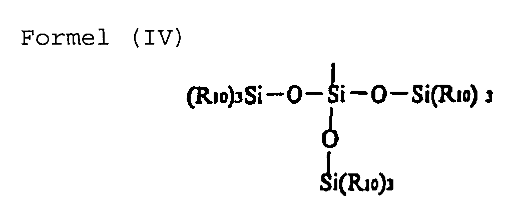 worin R₁₀ für eine Alkylgruppe mit 1-6 Kohlenstoffatomen steht; 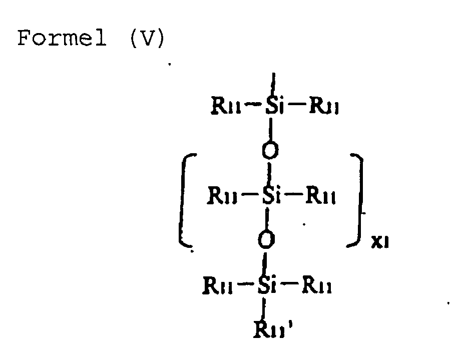 worin R₁₁ und R₁₁' jeweils für eine Alkylgruppe mit 1-6 Kohlenstoffatomen stehen und x1 für eine positive ganze Zahl steht; und 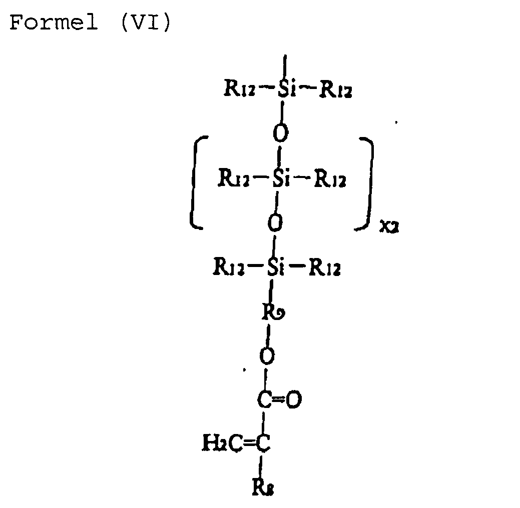 worin R₈ und R₉ wie in Formel (III) definiert sind; R₁₂ für eine Alkylgruppe mit 1-6 Kohlenstoffatomen steht und x2 für eine positive ganze Zahl steht, und
ein Monomer (D) der im folgenden angegebenen Formel (VII) : 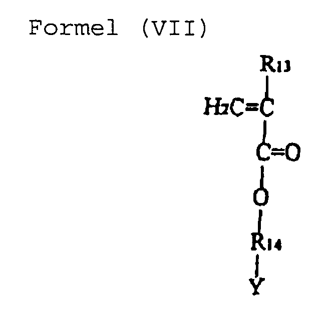 worin R₁₃ für ein Wasserstoffatom oder eine Methylgruppe steht; R₁₄ für eine Alkylengruppe mit 1-6 Kohlenstoffatomen steht; und Y für eine Gruppe steht, die durch -N⁺(R₁₅)₃ oder -N(R₁₅)₂ ausgedrückt wird, worin R₁₅ für eine Alkylgruppe mit 1-6 Kohlenstoffatomen steht, umfasst.

2. Zusammensetzung nach Anspruch 1, wobei der Prozentsatz des Monomers (A) 25 bis 99 Gew.-% in dem Copolymer beträgt.

3. Zusammensetzung nach Anspruch 1 oder 2, wobei der Prozentsatz des Monomers (B) 1 Gew.-% oder mehr in dem Copolymer beträgt.

4. Zusammensetzung nach einem der Ansprüche 1 bis 3, wobei der Prozentsatz des Monomers (C) 1 Gew.-% oder mehr in dem Copolymer beträgt.

5. Zusammensetzung nach Anspruch 1, wobei der Prozentsatz des Monomers (D) im Bereich von 1 bis 100 Gewichtsteilen in Bezug auf 100 Gewichtsteile einer Kombination von Monomer (A), Monomer (B) und Monomer (C) in dem Copolymer liegt.

6. Zusammensetzung nach einem der Ansprüche 1 bis 5, wobei R₃, R₄ und R₅ jeweils für eine Alkoxygruppe mit 1-6 Kohlenstoffatomen stehen.

7. Zusammensetzung nach einem der Ansprüche 1 bis 6, die ferner ein Pigment und/oder einen sauren Farbstoff umfasst.

8. Zusammensetzung nach einem der Ansprüche 1 bis 7, die ferner ein siliconisiertes Polysaccharid umfasst.

9. Copolymer, das ein Monomer (A) der Formel (I) gemäß der Definition in Anspruch 1, ein Monomer (B) der Formel (II) gemäß der Definition in Anspruch 1, ein Monomer (C) der Formel (III) gemäß der Definition in Anspruch 1, ein Monomer (D) der Formel (VII) gemäß der Definition in Anspruch 1 als konstitutive Monomere umfasst.

10. Copolymer nach Anspruch 9, wobei R₃, R₄ und R₅ jeweils für eine Alkoxygruppe mit 1-6 Kohlenstoffatomen stehen.

11. Verwendung der Zusammensetzung nach einem der Ansprüche 1 bis 8 zur Beschichtung einer Oberfläche.

12. Verwendung der Zusammensetzung nach einem der Ansprüche 1 bis 8 zur Modifizierung der Natur von Haar.

13. Verwendung der Zusammensetzung nach einem der Ansprüche 1 bis 8 als kosmetische Packung.

14. Verwendung der Zusammensetzung nach einem der Ansprüche 1 bis 8 als wasserabstoßendes Mittel.

15. Verwendung nach Anspruch 14, wobei der Prozentsatz des Monomers (A) 25 bis 85 Gew.-% in dem Copolymer beträgt.

16. Verwendung nach Anspruch 14 oder 15, wobei die Prozentsätze des Monomers (B) und des Monomers (C) 5 Gew.-% oder mehr bzw. 1 Gew.-% oder mehr in dem Copolymer betragen.

17. Verwendung einer Zusammensetzung nach einem der Ansprüche 1 bis 8 als Faserbehandlungszusammensetzung.

18. Verwendung nach Anspruch 17, wobei der Prozentsatz des Monomers (A) 25 bis 85 Gew.-% in dem Copolymer beträgt.

19. Verwendung nach Anspruch 17 oder 18, wobei die Prozentsätze des Monomers (B) und des Monomers (C) 5 Gew.-% oder mehr bzw. 1 Gew.-% oder mehr in dem Copolymer betragen.

20. Eine Beschichtung bildendes Verfahren, das die Stufe des Hydrolysierens der Zusammensetzung nach einem der Ansprüche 1 bis 8 auf einem zu behandelnden Material zur Vernetzung von Molekülen des Polymers umfasst.

21. Eine Beschichtung bildendes Verfahren nach Anspruch 20, wobei das zu behandelnde Material Menschenhaar oder -epidermis ist.

22. Eine Beschichtung bildendes Verfahren nach Anspruch 20, wobei das zu behandelnde Material Fasern, Papier oder Glas sind.

23. Verfahren für eine Packung, das die Stufen des Hydrolysierens der Zusammensetzung nach einem der Ansprüche 1 bis 6 auf Haut zur Vernetzung von Molekülen des Copolymers und des Ablösens der Zusammensetzung von der Haut nach dem Trocknen umfasst.

## Revendications

1. Composition comprenant un copolymère contenant des groupes silyles dans lequel chaque groupe silyle a au moins un groupe fonctionnel réactif lié à celui-ci,
dans laquelle ledit copolymère comprend le monomère (A) représenté par la formule (I) suivante : 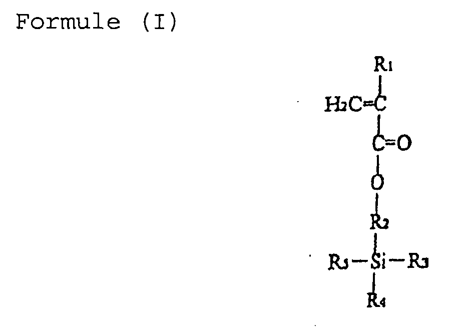 dans laquelle R₁ est un atome d'hydrogène ou un groupe méthyle ; R₂ est un groupe alkylène ayant de 1 à 6 atomes de carbone ; et R₃, R₄ et R₅ sont chacun un groupe fonctionnel réactif qui peut réticuler les molécules du copolymère par hydrolyse,
le monomère (B) représenté par la formule (II) suivante : 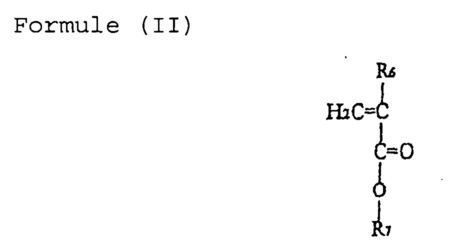 dans laquelle R₆ est un atome d'hydrogène ou un groupe méthyle ; et R₇ est un groupe alkyle ayant de 1 à 18 atomes de carbone,
le monomère (C) représenté par la formule (III) suivante : 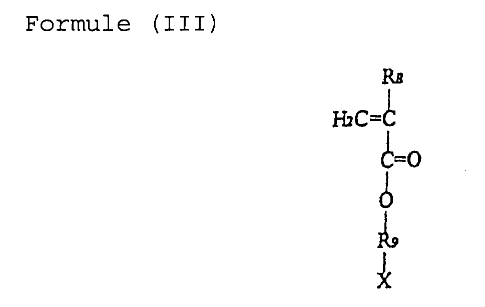 dans laquelle R₈ est un atome d'hydrogène ou un groupe méthyle ; R₉ est un groupe alkylène ayant de 1 à 6 atomes de carbone ; et X est un groupe représenté par l'une quelconque des formules (IV) à (VI) suivantes : 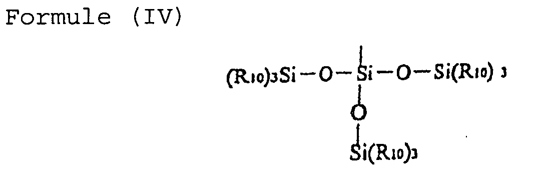 dans laquelle R₁₀ est un groupe alkyle ayant de 1 à 6 atomes de carbone ; 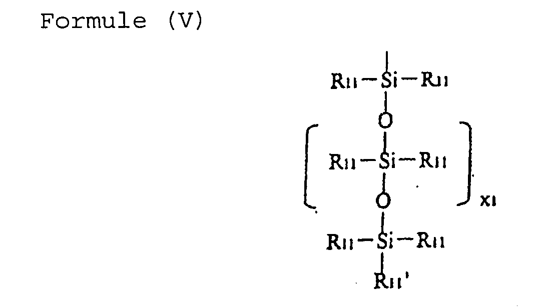 dans laquelle R₁₁ et R₁₁, sont chacun un groupe alkyle ayant de 1 à 6 atomes de carbone ; et x1 est un entier d'un nombre positif ; et 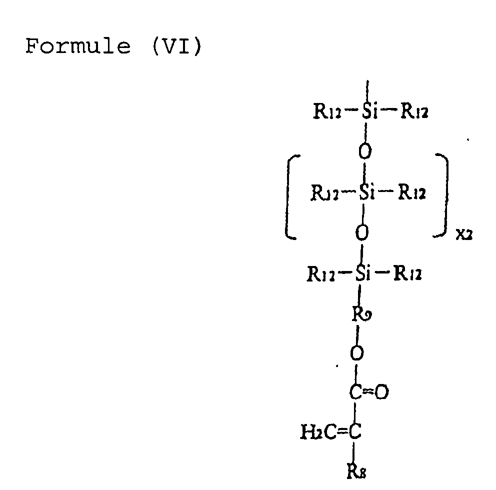 dans laquelle R₈ et R₉ sont identiques à ceux définis dans la formule (III) ; R₁₂ est un groupe alkyle ayant de 1 à 6 atomes de carbone ; et x2 est un entier d'un nombre positif, et
le monomère (D) représenté par la formule (VII) suivante : 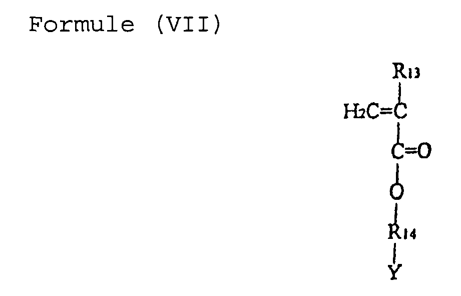 dans laquelle R₁₃ est un atome d'hydrogène ou un groupe méthyle ; R₁₄ est un groupe alkylène ayant de 1 à 6 atomes de carbone ; et Y est un groupe représenté par -N⁺(R₁₅)₃ ou -N(R₁₅)₂, où R₁₅ est un groupe alkyle ayant de 1 à 6 atomes de carbone.

2. Composition selon la revendication 1, dans laquelle le pourcentage de monomère (A) est de 25 à 99 % en poids dans le copolymère.

3. Composition selon la revendication 1 ou 2, dans laquelle le pourcentage de monomère (B) est de 1 % en poids ou plus dans le copolymère.

4. Composition selon l'une quelconque des revendications 1 à 3, dans laquelle le pourcentage de monomère (C) est de 1 % en poids ou plus dans le copolymère.

5. Composition selon la revendication 1, dans laquelle le pourcentage de monomère (D) est dans la gamme de 1 à 100 parties en poids pour 100 parties en poids d'une combinaison de monomère (A), de monomère (B) et de monomère (C) dans le copolymère.

6. Composition selon l'une quelconque des revendications 1 à 5, dans laquelle chacun parmi R₃, R₄ et R₅ est un groupe alcoxy ayant de 1 à 6 atomes de carbone.

7. Composition selon l'une quelconque des revendications 1 à 6, qui comprend en outre un pigment et/ou un colorant acide.

8. Composition selon l'une quelconque des revendications 1 à 7, qui comprend en outre un polysaccharide siliconé.

9. Copolymère comprenant le monomère (A) représenté par la formule (I) telle que définie dans la revendication 1, le monomère (B) représenté par la formule (II) telle que définie dans la revendication 1, le monomère (C) représenté par la formule (III) telle que définie dans la revendication 1, le monomère (D) représenté par la formule (VII) telle que définie dans la revendication 1, en tant que monomères constitutifs.

10. Copolymère selon la revendication 9, dans lequel chacun parmi R₃, R₄ et R₅ est un groupe alcoxy ayant de 1 à 6 atomes de carbone.

11. Utilisation de la composition selon l'une quelconque des revendications 1 à 8, pour revêtir une surface.

12. Utilisation de la composition selon l'une quelconque des revendications 1 à 8, pour modifier la nature de cheveux.

13. Utilisation d'une composition selon l'une quelconque des revendications 1 à 8 en tant que pack cosmétique.

14. Utilisation d'une composition selon l'une quelconque des revendications 1 à 8 en tant qu'hydrofuge.

15. Utilisation selon la revendication 14, dans laquelle le pourcentage de monomère (A) est de 25 à 85 % en poids dans le copolymère.

16. Utilisation selon la revendication 14 ou 15, dans laquelle les pourcentages de monomère (B) et de monomère (C) sont de 5 % en poids ou plus et de 1 % en poids ou plus dans le copolymère, respectivement.

17. Utilisation d'une composition selon l'une quelconque des revendications 1 à 8 en tant que composition pour traiter une fibre.

18. Utilisation selon la revendication 17, dans laquelle le pourcentage de monomère (A) est de 25 à 85 % en poids dans le copolymère.

19. Utilisation selon la revendication 17 ou 18, dans laquelle les pourcentages de monomère (B) et de monomère (C) sont de 5 % en poids ou plus et de 1 % en poids ou plus dans le copolymère, respectivement.

20. Procédé de formation de revêtement comprenant une étape consistant : à hydrolyser la composition selon l'une quelconque des revendications 1 à 8 sur un matériau à traiter pour réticuler les molécules du copolymère.

21. Procédé de formation de revêtement selon la revendication 20, dans lequel le matériau à traiter est des cheveux ou un épiderme humains.

22. Procédé de formation de revêtement selon la revendication 20, dans lequel le matériau à traiter est une fibre, un papier ou du verre.

23. Procédé pour un pack, comprenant les étapes consistant : à hydrolyser la composition selon l'une quelconque des revendications 1 à 6 sur la peau pour réticuler les molécules du copolymère, et à décoller la composition de la peau après séchage.
